# EUROPEAN PATENT APPLICATION

(11) **EP 3 786 161 A1**
(43) Date of publication of application: **03.03.2021**
(21) Application number: 19792044.0
(22) Date of filing: 23.04.2019
(51) Int. Cl.: C07D 403/14, A61K 31/517, A61P 9/00, A61P 25/28, A61P 29/00, A61P 31/00, A61P 35/00, A61P 37/00, A61P 43/00

(54) **CDK4/6 INHIBITOR AND PHARMACEUTICALLY ACCEPTABLE SALT AND POLYMORPH THEREOF AND USE THEREOF**

(30) Priority: 24.04.2018 CN 201810371832
(71) Applicant: Shanghai Haiyan Pharmaceutical Technology Co., Ltd, Shanghai 201203 (CN); Yangtze River Pharmaceutical Group Co., Ltd., Taizhou, Jiangsu 225321 (CN)
(72) Inventor: JIANG, Taotao, Shanghai 201203 (CN); LI, Jinjing, Shanghai 201203 (CN); ZHAO, Shuangni, Shanghai 201203 (CN); YAO, Xia, Shanghai 201203 (CN)
(74) Representative: Coles, Andrea Birgit
(86) International application number: PCT/CN2019/083970
(87) International publication number: WO 2019/206154

(57) **Abstract**

Provided are a CDK4/6 inhibitor, a pharmaceutically acceptable salt thereof and a polymorph thereof, and the use thereof. In particular, provided are a polymorph of 2-cyclopropyl-N-(5-((4-ethylpiperazin-1-yl)methyl)pyridin-2-yl)-3-isopropyl-3,8-dihydroimidazo[4',5',4,5]cyclopentadieno[1,2-d]pyrimidin-5-amine and a pharmaceutically acceptable salt thereof, and the use thereof. In addition, further disclosed are a pharmaceutical composition of the compound and the use thereof.

## Description

### TECHNICAL FIELD

The present disclosure belongs to the technical field of medicine. In particular, the present disclosure relates to a CDK4/6 inhibitor and pharmaceutically acceptable salts and polymorphs thereof and their applications, and the inhibitor is 2-cyclopropyl-N-(5-((4-ethylpiperazin-1-yl)methyl) pyridin-2-yl)-3-isopropyl-3,8-dihydroimidazo[4',5',4,5]cyclopenta[1,2-d]pyrimidin-5-amine.

### BACKGROUND

CDKs are a class of serine/threonine protein kinases. CDKs do not have kinase activity until they bind to a cyclin, and CDKs play a key role in the initiation of a cell cycle and in the conversion and regulation for respective phases of a cell cycle. CDK4/6 is an important cell cycle regulatory protein that phosphorylates the anti-oncogene protein Rb, releases the E2F transcription factor, and allows cells to successfully pass the cell cycle G1/S checkpoint, allowing the cell cycle to continue. A CDK4 single gene knockout mouse has both diabete and cell defect. A CDK6 single gene knockout mouse causes mild anemia due to defects in hematopoietic cell proliferation. While CDK4 and CDK6 (CDK4/6) double-gene knockout impaires the proliferative capacity of hematopoietic precursor cells, resulting in late embryonic death of the double-gene knockout mouse.The hyperactivation of CDK4/6-cyclin D/Rb signaling pathway has commonly been found in tumor cells. Various intracellular and extracellular mitotic signals stimulate cyclin D to highly express, regulate the interaction between CDK4/6 protein and cyclin D, and promote the localization and kinase activity of CDK4/6. Activated CDK4/6 inhibits the activity of Rb tumor suppressor protein by phosphorylation, dissociates the Rb-E2F complex, releases free E2F into the nucleus, regulates protein transcription, and initiates cell cycle progression. Hyperactivation of CDK4 has often been found in epithelial cell malignancies, and hyperactivation of CDK6 has often been often found in mesenchymal cell tumors such as sarcoma and hematological cancers. By constructing a tumor-bearing mouse model of breast cancer, it was found that tumor was developed in all the wild-type nude mice, but not in CDK4 knockout nude mice at all. By interfering with the expression of CDK4 using anti-CDK4 siRNA, it was found that tumor growth was significantly inhibited in nude mice. Selective CDK4/6 inhibitors can induce the block of G1 cell phase, thereby increasing the tolerance of hematopoietic stem/progenitor cells to DNA damaging agents such as IR, and effectively reducing the various hematopoietic toxicities induced by radiation, including myelosuppression, neutropenia, leukopenia, anemia, etc.

In recent years, major companies have identified and found a series of inhibitors such as palbociclib from Pfizer, Abemaciclib from Eli Lilly, which selectively inhibit CDK4 and CDK6, and are used to treat diseases such as cancers, cardiovascular disorders, inflammation, etc. In addition, a number of domestic companies also have patent disclosures. Patent applications of selective inhibition of CDK4 and CDK6 include WO2014183520, WO2015101293, WO2015180642, WO2016014904, WO2016015597, etc. Although these small molecule CDK inhibitors currently have certain advantages in clinical practice, they also have their own shortcomings, for example, palbociclib has relatively great neutrophil toxicity. It is generally believed that inhibition of CDK4 can inhibit the growth of tumor cells, while CDK6 is highly expressed in the blood system and functions to regulate the growth of hematopoietic cells, therefore, the inhibition of CDK6 may cause hematological toxicity, such as neutrophils reduction, red blood cell reduction, etc. Palbociclib has the same inhibition of both CDK4 and CDK6, with enzyme activities of 10 nm and 10 nm, respectively, to which the toxicity of Palbociclib should be related. While Abemaciclib has a stronger inhibition on CDK4 than on CDK6; the weak CDK6 inhibitor causes low hematological toxicity. Since the homology between CDK4 and CDK6 is very high, i.e., about 70%, the development of selective CDK4/6 inhibitors, especially CDK4 inhibitors, is a great challenge.

In addition, brain metastases occur in a significant proportion of advanced cancer patients, which is particularly prominent in lung cancer, breast cancer, and melanoma; for these patients, the existing treatment method will have very poor effect, this mainly because most of the drugs cannot enter the blood-brain barrier. Therefore, if selective CDK4/6 inhibitors which have unique pharmacokinetic characteristics, can effectively penetrate the blood-brain barrier, and have significant efficacy for patients with brain tumors or brain metastases with the current clinical significant needs can be developed, they will have important clinical significance and broad market prospects. In order to achieve a better therapeutic effect on tumors and better meet market demands, we hope to develop a new generation of highly efficient and low-toxic selective CDK4 and CDK6 inhibitors.The present disclosure develops various salt forms and crystalline forms of the CDK4/6 inhibitor on the basis of the foregoing work, which is helpful for further drug development.

### SUMMARY OF THE INVENTION

The object of the present disclosure is to provide pharmaceutically acceptable salts of a CDK4/6 inhibitor and polymorphs and applications thereof.

In a first aspect of the present disclosure, a pharmaceutically acceptable salt of a compound of formula X, a polymorph of the compound of formula X and a polymorph of pharmaceutically acceptable salt of the compound of formula X, is provided:

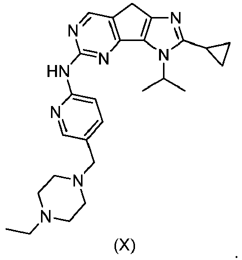

In another preferred embodiment, the pharmaceutically acceptable salt is selected from the group consisting of hydrochloride, sulfate, hydrobromide, phosphate, methanesulfonate, maleate, L-tartrate, citrate, fumarate, and formate.

In another preferred embodiment, the pharmaceutically acceptable salt of the compound of formula X or the polymorph of the compound of formula X and a polymorph of pharmaceutically acceptable salt of the compound of formula X is in an anhydrous form, hydrate form or solvate form.

In another preferred embodiment, the pharmaceutically acceptable salt is selected from the group consisting of maleate, hydrochloride, and formate.

In another preferred embodiment, the pharmaceutically acceptable salt is maleate, and the molar ratio of maleic acid to the compound of formula X is (0.8-2.1): 1, preferably (1.1-1.2): 1.

In another preferred embodiment, the pharmaceutically acceptable salt is hydrochloride, and the molar ratio of hydrochloric acid to the compound of formula X is (0.8-2.1): 1, preferably (1.1-1.2): 1.

In another preferred embodiment, the polymorph is A crystalline form of the maleate of compound of formula X, i.e. crystal form A, the X-ray powder diffraction pattern of which has peaks at diffraction angles 2θ(°) values of the following group A1: 4.47±0.2, 8.93±0.2, 13.41±0.2, 13.98±0.2, 15.77±0.2, 16.52±0.2, 17.18±0.2, 18.06±0.2, 18.61±0.2, 19.16±0.2, 21.50±0.2, 22.26±0.2, 23.43±0.2, and 23.84±0.2 .

In another preferred embodiment, the X-ray powder diffraction pattern of the crystal form A further has peaks at 2 or more than 2 of diffraction angles 2θ(°) values selected from the following group A2: 14.97±0.2, 15.95±0.2, 20.27±0.2, 20.90±0.2, 24.08±0.2, 24.83±0.2, 26.20±0.2, and 30.38±0.2.

In another preferred embodiment, the X-ray powder diffraction pattern of the crystal form A further has peaks at 2 or more than 2 of diffraction angles 2θ (°) values selected from the following group A3: 10.42±0.2, 11.11±0.2, 12.78±0.2, 21.96±0.2, 22.77±0.2, 27.03±0.2, 27.88±0.2, 28.60±0.2, 29.06±0.2, 31.64±0.2, 32.29±0.2, and 35.91±0.2.

In another preferred embodiment, the X-ray powder diffraction pattern of the crystal form A has peaks at 6 or more or all (such as 6, 7, 8, 9, 10, 11, 12, 13, 14, 15, etc.) of diffraction angles 2θ (°) values selected from the groups A1, A2 and A3.

In another preferred embodiment, the X-ray powder diffraction pattern of the crystal form A has peaks at 2θ(°) values shown in table A1, and the relative intensity of each peak is as shown in table A1:

**Table A1**

| 2θ(°) | I/I₀ | 2θ(°) | I/I₀ | 2θ(°) | I/I₀ |
|---|---|---|---|---|---|
| 4.47 | VS | 8.93 | S | 10.42 | W |
| 11.11 | W | 12.78 | W | 13.41 | VS |
| 13.98 | S | 14.97 | 22 | 15.77 | S |
| 15.95 | M | 16.52 | S | 17.18 | S |
| 18.06 | VS | 18.61 | VS | 19.16 | S |
| 20.27 | M | 20.90 | M | 21.50 | S |
| 21.96 | W | 22.26 | VS | 22.77 | W |
| 23.43 | VS | 23.84 | S | 24.08 | M |
| 24.83 | M | 26.20 | M | 27.03 | W |
| 27.88 | W | 28.60 | W | 29.06 | W |
| 30.38 | M | 31.64 | W | 32.29 | W |
| 35.91 | W | | | | |

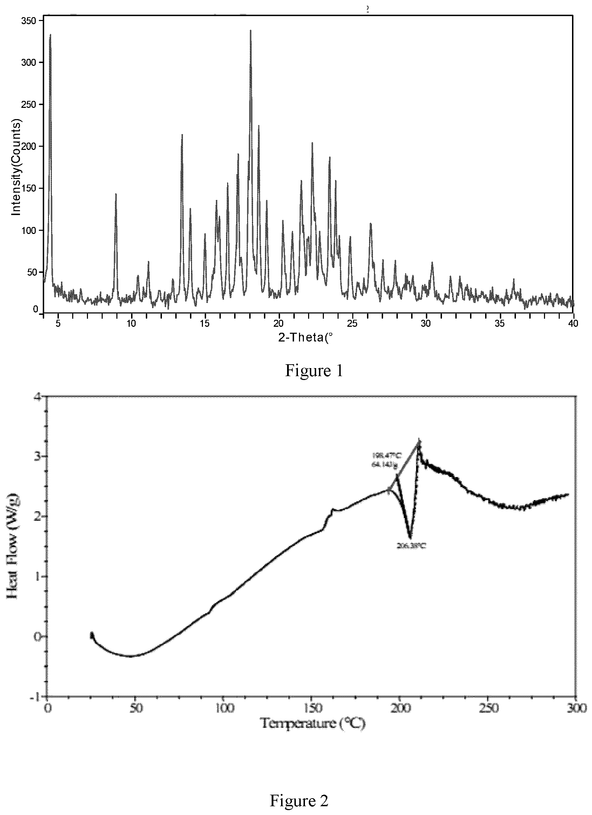

In another preferred embodiment, the X-ray powder diffraction pattern of crystal form A is substantially as characterized in Figure 1.

In another preferred embodiment, in the crystal form A, the molar ratio of maleic acid to the compound of formula X is (0.8-2.1): 1, preferably (1.0-1.2): 1, more preferably 1.2: 1.

In another preferred embodiment, the crystal form A is in an anhydrous form.

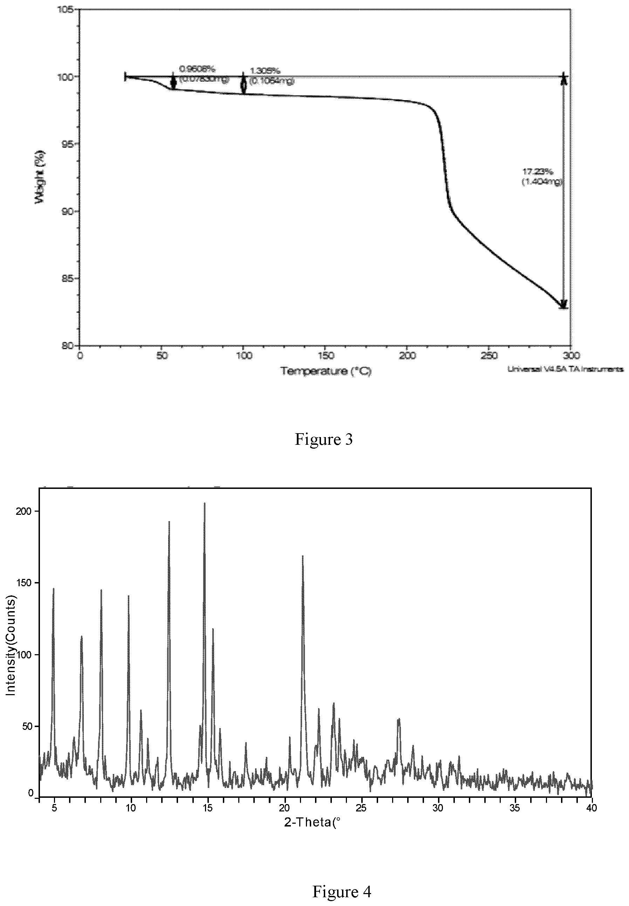

In another preferred embodiment, the crystal form A further has one or more features selected from the following group:
(i) In the differential scanning calorimetry analysis spectrum, the initial temperature is 193 ± 2 °C; preferably, the differential scanning calorimetry analysis spectrum is substantially as characterized in Figure 2;
(ii) the thermogravimetric analysis spectrum is substantially as characterized in Figure 3;
(iii) The melting point of the crystal form A is 193-211 °C, preferably 205-207 °C.

In another preferred embodiment, the polymorph is B-1 crystalline form of the hydrochloride of compound of formula X, i.e. crystal form B-1, the X-ray powder diffraction pattern of which has peaks at diffraction angles 2θ(°) values of the following group B-1-1: 4.93±0.2, 6.78±0.2, 8.04±0.2, 9.82±0.2, 12.46±0.2, 14.75±0.2, 15.32±0.2, and 21.17±0.2.

In another preferred embodiment, the X-ray powder diffraction pattern of the crystal form B-1 further has peaks at 2 or more than 2 of diffraction angles 2θ (°) values selected from the following group B-1-2: 10.63±0.2, 14.48±0.2, 15.78±0.2, 22.21±0.2, 23.19±0.2, 23.56±0.2, and 27.42±0.2.

In another preferred embodiment, the X-ray powder diffraction pattern of the crystal form B-1 further has peaks at 2 or more than 2 of diffraction angles 2θ (°) values selected from the following group B-1-3: 6.30±0.2, 11.09±0.2, 11.69±0.2, 17.46±0.2, 18.80±0.2, 20.32±0.2, and 28.32±0.2.

In another preferred embodiment, the X-ray powder diffraction pattern of the crystal form B-1 has peaks at 6 or more or all (such as 6, 7, 8, 9, 10, 11, 12, 13, 14, 15, etc.) of diffraction angles 29 (°) values selected from the groups B-1-1, B-1-2 and B-1-3.

In another preferred embodiment, the X-ray powder diffraction pattern of the crystal form B-1 has peaks at 2θ(°) values shown in table (B-1), and the relative intensity of each peak is as shown in table (B-1):

**Table B-1**

| 2θ(°) | I/I₀ | 2θ(°) | I/I₀ | 2θ(°) | I/I₀ |
|---|---|---|---|---|---|
| 4.93 | VS | 6.30 | W | 6.78 | S |
| 8.04 | VS | 9.82 | VS | 10.63 | M |
| 11.09 | W | 11.69 | W | 12.46 | VS |
| 14.48 | M | 14.75 | VS | 15.32 | VS |
| 15.78 | M | 17.46 | W | 18.80 | W |
| 20.32 | W | 21.17 | VS | 22.21 | M |
| 23.19 | M | 23.56 | M | 27.42 | M |
| 28.32 | W | | | | |

In another preferred embodiment, the X-ray powder diffraction pattern of crystal form B-1 is substantially as characterized in Figure 4.

In another preferred embodiment, in the crystal form B-1, the molar ratio of hydrochloric acid to the compound of formula X is (0.8-2.1): 1, preferably (1.0-1.2): 1, more preferably 1.2: 1.

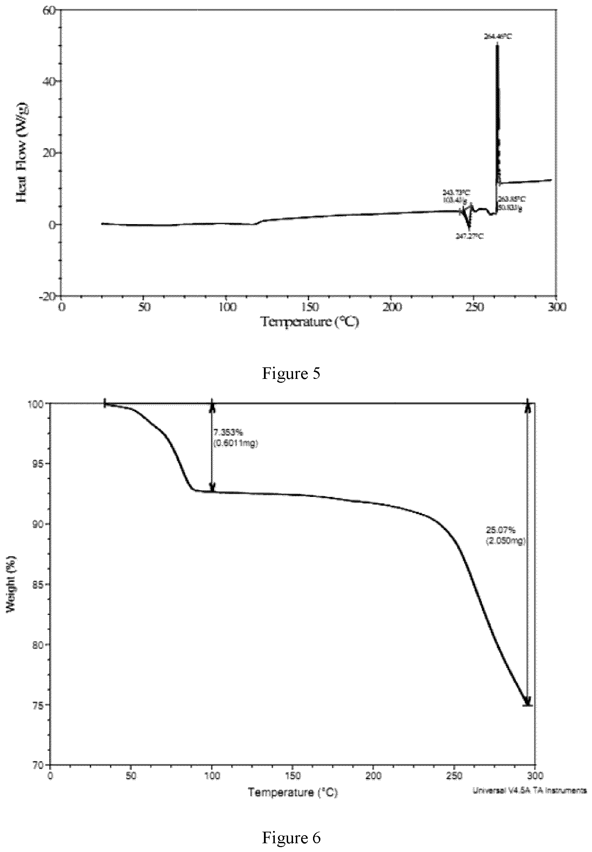

In another preferred embodiment, the crystal form B-1 further has one or more features selected from the following group:
(i) In the differential scanning calorimetry analysis spectrum, the initial temperature are 240±2 °C and 263±2°C; preferably, the differential scanning calorimetry analysis spectrum is substantially as characterized in Figure 5;
(ii) the thermogravimetric analysis spectrum is substantially as characterized in Figure 6;
(iii) The weight loss of the crystal form B-1 is about 7.4% under 50 °C- 90 °C. According to the calculation, the reason should be the removal of HCl from the compound, which compound is unstable at high temperature (above 50 °C).

In another preferred embodiment, the polymorph is B-2 crystalline form of the hydrochloride of compound of formula X, i.e. crystal form B-2, the X-ray powder diffraction pattern of which has peaks at diffraction angles 2θ(°) values of the following group B-2-1: 4.56±0.2, 11.41±0.2, and 13.60±0.2.

In another preferred embodiment, the X-ray powder diffraction pattern of the crystal form B-2 further has peaks at diffraction angles 2θ (°) values of the following group B-2-2: 5.72±0.2, 9.06±0.2, 17.72±0.2, 22.92±0.2, and 23.71±0.2.

In another preferred embodiment, the X-ray powder diffraction pattern of the crystal form B-2 has peaks at 6 or more or all (such as 6, 7, 8, etc.) of 2θ (°) values selected from the groups B-2-1 and B-2-2.

In another preferred embodiment, the X-ray powder diffraction pattern of the crystal form B-2 has peaks at 2θ(°) values shown in table (B-2), and the relative intensity of each peak is as shown in table (B-2):

**Table (B-2)**

| 2θ(°) | I/I₀ | 2θ(°) | I/I₀ | 2θ(°) | I/I₀ |
|---|---|---|---|---|---|
| 4.56 | VS | 5.72 | M | 9.06 | M |
| 11.41 | S | 13.60 | S | 17.72 | M |
| 22.92 | M | 23.71 | M | | |

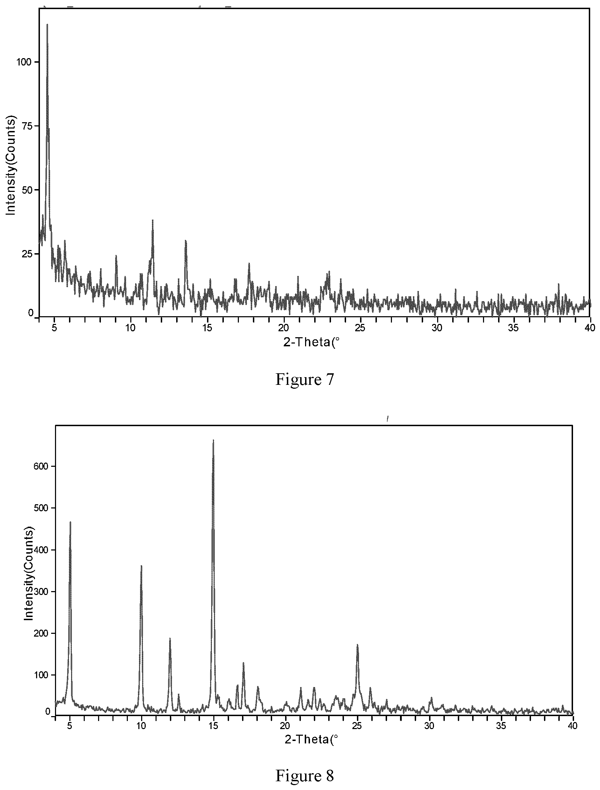

In another preferred embodiment, the X-ray powder diffraction pattern of crystal form B-2 is substantially as characterized in Figure 7.

In another preferred embodiment, the polymorph is B-3 crystalline form of the hydrochloride of compound of formula X, i.e. crystal form B-3, the X-ray powder diffraction pattern of which has peaks at diffraction angles 2θ(°) values of the following group B-3-1: 5.03±0.2, 9.97±0.2, and 14.96±0.2.

In another preferred embodiment, the X-ray powder diffraction pattern of the crystal form B-3 further has peaks at diffraction angles 2θ (°) values of the following group B-3-2: 11.98±0.2, 17.07±0.2, and 25.00±0.2.

In another preferred embodiment, the X-ray powder diffraction pattern of the crystal form B-3 further has peaks at 2 or more than 2 of diffraction angles 2θ (°) values of the following group B-3-3: 12.59±0.2, 16.06±0.2, 16.64±0.2, 18.07±0.2, 20.05±0.2, 21.05±0.2, 21.98±0.2, 22.40±0.2, 23.50±0.2, 24.04±0.2, 25.90±0.2, 27.03±0.2, and 30.12±0.2.

In another preferred embodiment, the X-ray powder diffraction pattern of the crystal form B-3 has peaks at 6 or more or all (such as 6, 7, 8, 9, 10, 11, 12, 13, 14, 15, etc.) of 2θ (°) values selected from the groups B-3-1, B-3-2 and B-3-3.

In another preferred embodiment, the X-ray powder diffraction pattern of the crystal form B-3 has peaks at 2θ(°) values shown in table(B-3), and the relative intensity of each peak is as shown in table (B-3):

**Table (B-3)**

| 2θ(°) | I/I₀ | 2θ(°) | I/I₀ | 2θ(°) | I/I₀ |
|---|---|---|---|---|---|
| 5.03 | VS | 9.97 | VS | 11.98 | M |
| 12.59 | W | 14.96 | VS | 16.06 | W |
| 16.64 | W | 17.07 | M | 18.07 | W |
| 20.05 | W | 21.05 | W | 21.98 | W |
| 22.40 | W | 23.50 | W | 24.04 | W |
| 25.00 | M | 25.90 | W | 27.03 | W |
| 30.12 | W | | | | |

In another preferred embodiment, the X-ray powder diffraction pattern of crystal form B-3 is substantially as characterized in Figure 8.

In another preferred embodiment, the polymorph is C-1 crystalline form of the sulfate of compound of formula X, i.e. crystal form C-1, the X-ray powder diffraction pattern of which has peaks at diffraction angles 29(°) values of the following group C-1-1: 9.13±0.2, 9.71±0.2, 10.50±0.2, 11.19±0.2, 13.72±0.2, 13.94±0.2, 15.70±0.2, 16.79±0.2, 22.46±0.2, and 23.87±0.2.

In another preferred embodiment, the X-ray powder diffraction pattern of the crystal form C-1 further has peaks at 2 or more than 2 of diffraction angles 2θ (°) values selected from the following group C-1-2: 4.60±0.2, 12.35±0.2, 15.26±0.2, 18.23±0.2, 19.40±0.2, 25.56±0.2, 26.11±0.2, 27.68±0.2, and 28.43±0.2.

In another preferred embodiment, the X-ray powder diffraction pattern of the crystal form C-1 further has peaks at 2 or more than 2 of diffraction angles 2θ (°) values selected from the following group C-1-3: 14.78±0.2, and 21.50±0.2.

In another preferred embodiment, the X-ray powder diffraction pattern of the crystal form C-1 has peaks at 6 or more or all (such as 6, 7, 8, 9, 10, 11, 12, 13, 14, 15, etc.) of 2θ (°) values selected from the groups C-1-1, C-1-2 and C-1-3.

In another preferred embodiment, the X-ray powder diffraction pattern of the crystal form C-1 has peaks at 2θ(°) values shown in table (C-1), and the relative intensity of each peak is as shown in table (C-1):

**Table C-1**

| 2θ(°) | I/I₀ | 2θ(°) | I/I₀ | 2θ(°) | I/I₀ |
|---|---|---|---|---|---|
| 4.60 | M | 9.13 | S | 9.71 | S |
| 10.50 | S | 11.19 | S | 12.35 | M |
| 13.72 | VS | 13.94 | S | 14.78 | W |
| 15.26 | M | 15.70 | S | 16.79 | S |
| 18.23 | M | 19.40 | M | 21.50 | W |
| 22.46 | VS | 23.87 | S | 25.56 | M |
| 26.11 | M | 27.68 | M | 28.43 | M |

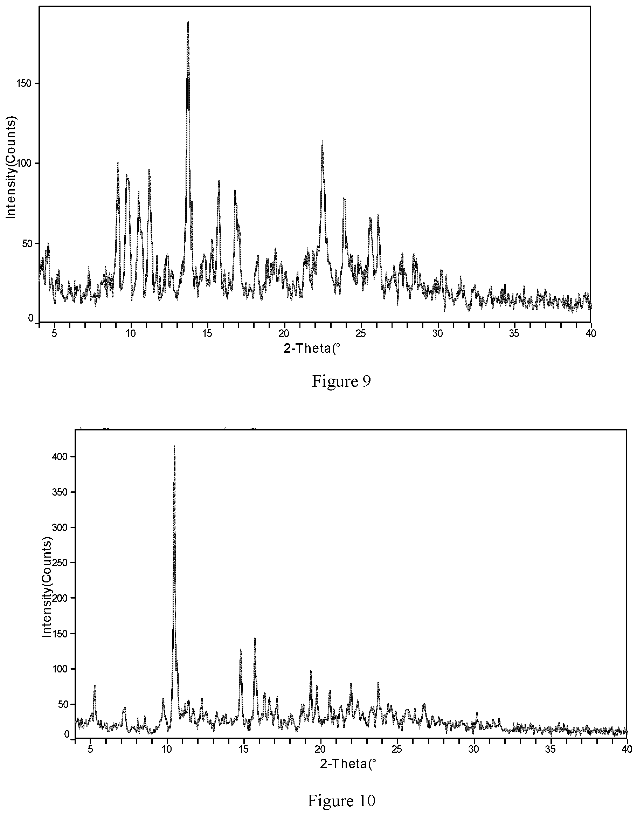

In another preferred embodiment, the X-ray powder diffraction pattern of crystal form C-1 is substantially as characterized in Figure 9.

In another preferred embodiment, the polymorph is C-2 crystalline form of the sulfate of compound of formula X, i.e. crystal form C-2, the X-ray powder diffraction pattern of which has peaks at diffraction angles 2θ(°) values of the following group C-2-1: 10.47±0.2, 14.78±0.2, and 15.72±0.2.

In another preferred embodiment, the X-ray powder diffraction pattern of the crystal form C-2 further has peaks at diffraction angles 2θ (°) values of the following group C-2-2: 5.29±0.2, 19.36±0.2, 19.75±0.2, 20.58±0.2, and 21.96±0.2.

In another preferred embodiment, the X-ray powder diffraction pattern of the crystal form C-2 further has peaks at 2 or more than 2 of diffraction angles 2θ (°) values selected from the following group C-2-3: 7.21±0.2, 9.74±0.2, 11.37±0.2, 12.25±0.2, 16.34±0.2, 16.67±0.2, 17.14±0.2, 18.89±0.2, 21.30±0.2, 22.40±0.2, 23.28±0.2, 23.75±0.2, 24.59±0.2, and 26.73±0.2.

In another preferred embodiment, the X-ray powder diffraction pattern of the crystal form C-2 has peaks at 6 or more or all (such as 6, 7, 8, 9, 10, 11, 12, 13, 14, 15, etc.) of 2θ (°) values selected from the groups C-2-1, C-2-2 and C-2-3.

In another preferred embodiment, the X-ray powder diffraction pattern of the crystal form C-2 has peaks at 2θ(°) values shown in table(C-2), and the relative intensity of each peak is as shown in table (C-2):

**Table (C-2)**

| 2θ(°) | I/I₀ | 2θ(°) | I/I₀ | 2θ(°) | I/I₀ |
|---|---|---|---|---|---|
| 5.29 | M | 7.21 | W | 9.74 | W |
| 10.47 | VS | 11.37 | W | 12.25 | W |
| 14.78 | S | 15.72 | S | 16.34 | W |
| 16.67 | W | 17.14 | W | 18.89 | W |
| 19.36 | M | 19.75 | M | 20.58 | M |
| 21.30 | W | 21.96 | M | 22.40 | W |
| 23.28 | W | 23.75 | W | 24.59 | W |
| 26.73 | W | | | | |

In another preferred embodiment, the X-ray powder diffraction pattern of crystal form C-2 is substantially as characterized in Figure 10.

In another preferred embodiment, the polymorph is D crystalline form of the hydrobromide of compound of formula X, i.e. crystal form D, the X-ray powder diffraction pattern of which has peaks at diffraction angles 2θ(°) values of the following group D1: 7.99±0.2, 9.74±0.2, 10.53±0.2, 12.37±0.2, 14.64±0.2, 15.21±0.2, 21.04±0.2, 22.11±0.2, 23.03±0.2, 23.38±0.2, 24.45±0.2, and 27.22±0.2.

In another preferred embodiment, the X-ray powder diffraction pattern of the crystal form D further has peaks at 2 or more than 2 of diffraction angles 2θ (°) values selected from the following group D2: 14.42±0.2, 15.76±0.2, 17.30±0.2, 18.09±0.2, 18.74±0.2, 20.29±0.2, 26.48±0.2, 28.10±0.2, 28.74±0.2, 29.86±0.2, 30.64±0.2, 31.11±0.2, and 32.11±0.2.

In another preferred embodiment, the X-ray powder diffraction pattern of the crystal form D further has peaks at diffraction angles 2θ (°) values selected from the following group D3: 29.09±0.2.

In another preferred embodiment, the X-ray powder diffraction pattern of the crystal form D has peaks at 6 or more or all (such as 6, 7, 8, 9, 10, 11, 12, 13, 14, 15, etc.) of 2θ (°) values selected from the groups D1, D2 and D3.

In another preferred embodiment, the X-ray powder diffraction pattern of the crystal form D has peaks at 2θ(°) values shown in table D1, and the relative intensity of each peak is as shown in table D1:

**Table D1**

| 2θ(°) | I/I₀ | 2θ(°) | I/I₀ | 2θ(°) | I/I₀ |
|---|---|---|---|---|---|
| 7.99 | S | 9.74 | S | 10.53 | S |
| 12.37 | VS | 14.42 | M | 14.64 | S |
| 15.21 | S | 15.76 | M | 17.30 | M |
| 18.09 | M | 18.74 | M | 20.29 | M |
| 21.04 | VS | 22.11 | S | 23.03 | S |
| 23.38 | S | 24.45 | S | 26.48 | M |
| 27.22 | VS | 28.10 | M | 28.74 | M |
| 29.09 | W | 29.86 | M | 30.64 | M |
| 31.11 | M | 32.11 | M | | |

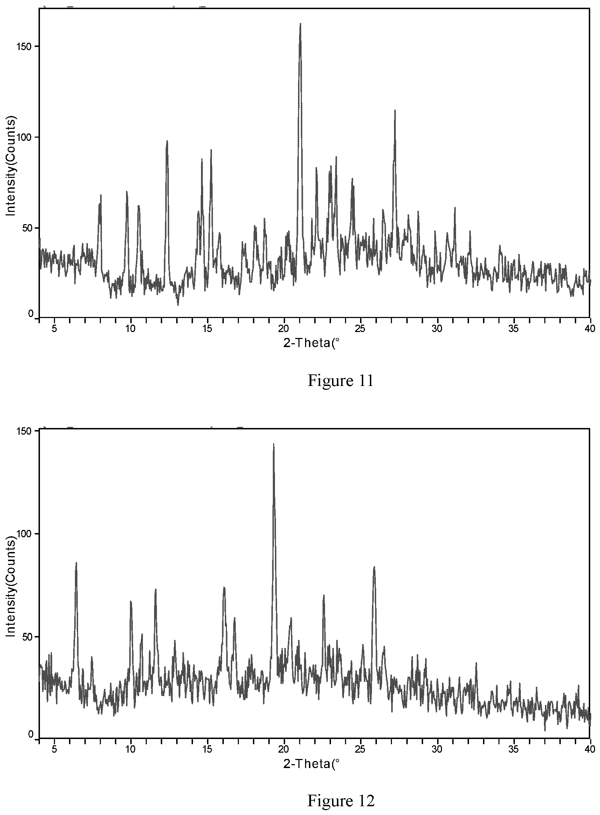

In another preferred embodiment, the X-ray powder diffraction pattern of crystal form D is substantially as characterized in Figure 11.

In another preferred embodiment, the polymorph is E crystalline form of the L-tartrate of compound of formula X, i.e. crystal form E, the X-ray powder diffraction pattern of which has peaks at diffraction angles 2θ(°) values of the following group E1: 6.43±0.2, 10.02±0.2, 11.63±0.2, 16.07±0.2, 19.33±0.2, 22.59±0.2, and 25.88±0.2.

In another preferred embodiment, the X-ray powder diffraction pattern of the crystal form E further has peaks at 2 or more than 2 of diffraction angles 2θ (°) values selected from the following group E2: 7.46±0.2, 10.69±0.2, 12.88±0.2, 16.76±0.2, 20.42±0.2, 25.13±0.2, and 26.52±0.2.

In another preferred embodiment, the X-ray powder diffraction pattern of the crystal form E has peaks at 6 or more or all (such as 6, 7, 8, 9, 10, 11, 12, 13, 14, etc.) of 2θ (°) values selected from the groups E1, E2.

In another preferred embodiment, the X-ray powder diffraction pattern of the crystal form E has peaks at 2θ(°) values shown in table E1, and the relative intensity of each peak is as shown in table E1:

**Table E1**

| 2θ(°) | I/I₀ | 2θ(°) | I/I₀ | 2θ(°) | I/I₀ |
|---|---|---|---|---|---|
| 6.43 | VS | 7.46 | M | 10.02 | S |
| 10.69 | M | 11.63 | S | 12.88 | M |
| 16.07 | S | 16.76 | M | 19.33 | VS |
| 20.42 | M | 22.59 | S | 25.13 | M |
| 25.88 | S | 26.52 | M | | |

In another preferred embodiment, the X-ray powder diffraction pattern of crystal form E is substantially as characterized in Figure 12.

In another preferred embodiment, the polymorph is F crystalline form of the phosphate of compound of formula X, i.e. crystal form F, the X-ray powder diffraction pattern of which has peaks at diffraction angles 2θ(°) values of the following group F1: 12.03±0.2, 17.26±0.2, and 19.65±0.2.

In another preferred embodiment, the X-ray powder diffraction pattern of the crystal form F further has peaks at diffraction angles 2θ (°) values of the following group F2: 5.99±0.2, 18.20±0.2, and 21.01±0.2.

In another preferred embodiment, the X-ray powder diffraction pattern of the crystal form F further has peaks at 2 or more than 2 of diffraction angles 2θ (°) values selected from the following group F3: 8.59±0.2, 13.41±0.2, 13.69±0.2, 14.21±0.2, 15.57±0.2, 19.15±0.2, 20.34±0.2, 21.70±0.2, 21.99±0.2, 22.98±0.2, 23.59±0.2, 25.01±0.2, 25.52±0.2, 26.74±0.2, and 34.64±0.2.

In another preferred embodiment, the X-ray powder diffraction pattern of the crystal form F has peaks at 6 or more or all (such as 6, 7, 8, 9, 10, 11, 12, 13, 14, 15, etc.) of 2θ (°) values selected from the groups F1, F2 and F3.

In another preferred embodiment, the X-ray powder diffraction pattern of the crystal form F has peaks at 2θ(°) values shown in table F1, and the relative intensity of each peak is as shown in table F1:

**Table F1**

| 2θ(°) | I/I₀ | 2θ(°) | I/I₀ | 2θ(°) | I/I₀ |
|---|---|---|---|---|---|
| 5.99 | M | 8.59 | W | 12.03 | VS |
| 13.41 | W | 13.69 | W | 14.21 | W |
| 15.57 | W | 17.26 | S | 18.20 | M |
| 19.15 | W | 19.65 | S | 20.34 | W |
| 21.01 | M | 21.70 | W | 21.99 | W |
| 22.98 | W | 23.59 | W | 25.01 | W |
| 25.52 | W | 26.74 | W | 34.64 | W |

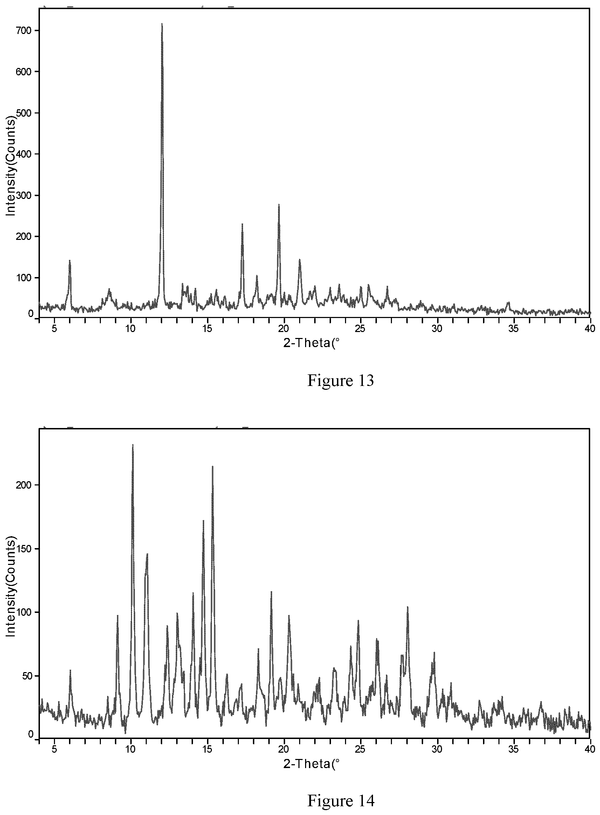

In another preferred embodiment, the X-ray powder diffraction pattern of crystal form F is substantially as characterized in Figure 13.

In another preferred embodiment, the polymorph is G crystalline form of the citrate of compound of formula X, i.e. crystal form G, the X-ray powder diffraction pattern of which has peaks at diffraction angles 2θ(°) values of the following group G1: 9.13±0.2, 10.13±0.2, 11.06±0.2, 12.38±0.2, 13.04±0.2, 14.07±0.2, 14.72±0.2, 15.33±0.2, 19.16±0.2, 20.31±0.2, 24.83±0.2, and 28.04±0.2.

In another preferred embodiment, the X-ray powder diffraction pattern of the crystal form G further has peaks at 2 or more than 2 of diffraction angles 2θ (°) values selected from the following group G2: 6.05±0.2, 16.25±0.2, 17.18±0.2, 18.31±0.2, 20.58±0.2, 22.29±0.2, 23.26±0.2, 24.35±0.2, 26.06±0.2, 27.67±0.2, and 29.77±0.2.

In another preferred embodiment, the X-ray powder diffraction pattern of the crystal form G further has peaks at 2 or more than 2 of diffraction angles 2θ (°) values selected from the following group G3: 8.48±0.2, 13.43±0.2, 19.74±0.2, 20.92±0.2, 26.66±0.2, 30.85±0.2, 32.75±0.2, and 34.21±0.2.

In another preferred embodiment, the X-ray powder diffraction pattern of the crystal form G has peaks at 6 or more or all (such as 6, 7, 8, 9, 10, 11, 12, 13, 14, 15, etc.) of 2θ (°) values selected from the groups G1, G2 and G3.

In another preferred embodiment, the X-ray powder diffraction pattern of the crystal form G has peaks at 2θ(°) values shown in table G1, and the relative intensity of each peak is as shown in table G1:

**Table G1**

| 2θ(°) | I/I₀ | 2θ(°) | I/I₀ | 2θ(°) | I/I₀ |
|---|---|---|---|---|---|
| 6.05 | M | 8.48 | w | 9.13 | S |
| 10.13 | VS | 11.06 | VS | 12.38 | S |
| 13.04 | S | 13.43 | w | 14.07 | S |
| 14.72 | VS | 15.33 | VS | 16.25 | M |
| 17.18 | M | 18.31 | M | 19.16 | S |
| 19.74 | w | 20.31 | S | 20.58 | M |
| 20.92 | w | 22.29 | M | 23.26 | M |
| 24.35 | M | 24.83 | S | 26.06 | M |
| 26.66 | w | 27.67 | M | 28.04 | S |
| 29.77 | M | 30.85 | w | 32.75 | w |
| 34.21 | w | | | | |

In another preferred embodiment, the X-ray powder diffraction pattern of crystal form G is substantially as characterized in Figure 14.

In another preferred embodiment, the polymorph is H-1 crystalline form of the fumarate of compound of formula X, i.e. crystal form H-1, the X-ray powder diffraction pattern of which has peaks at diffraction angles 2θ(°) values of the following group H-1-1: 5.37±0.2, 10.74±0.2, 17.67±0.2, 19.08±0.2, 19.35±0.2, 20.11±0.2, 21.25±0.2, and 22.84±0.2.

In another preferred embodiment, the X-ray powder diffraction pattern of the crystal form H-1 further has peaks at 2 or more than 2 of diffraction angles 2θ (°) values selected from the following group H-1-2: 8.83±0.2, 11.60±0.2, 12.33±0.2, 13.57±0.2, 15.38±0.2, 16.06±0.2, 16.56±0.2, 17.08±0.2, 18.41±0.2, 19.66±0.2, 21.74±0.2, 23.59±0.2, 24.19±0.2, and 29.98±0.2.

In another preferred embodiment, the X-ray powder diffraction pattern of the crystal form H-1 further has peaks at 2 or more than 2 of diffraction angles 2θ (°) values selected from the following group H-1-3: 12.03±0.2, 14.47±0.2, 17.33±0.2, 25.56±0.2, 26.49±0.2, 28.56±0.2, 29.10±0.2, 33.12±0.2.

In another preferred embodiment, the X-ray powder diffraction pattern of the crystal form H-1 has peaks at 6 or more or all (such as 6, 7, 8, 9, 10, 11, 12, 13, 14, 15, etc.) of 2θ (°) values selected from the groups H-1-1, H-1-2 and H-1-3.

In another preferred embodiment, the X-ray powder diffraction pattern of the crystal form H-1 has peaks at 2θ(°) values shown in table (H-1), and the relative intensity of each peak is as shown in table (H-1):

**Table H-1**

| 2θ(°) | I/I₀ | 2θ(°) | I/I₀ | 2θ(°) | I/I₀ |
|---|---|---|---|---|---|
| 5.37 | S | 8.83 | M | 10.74 | VS |
| 11.60 | M | 12.03 | w | 12.33 | M |
| 13.57 | M | 14.47 | w | 15.38 | M |
| 16.06 | M | 16.56 | M | 17.08 | M |
| 17.33 | W | 17.67 | S | 18.41 | M |
| 19.08 | S | 19.35 | S | 19.66 | M |
| 20.11 | S | 21.25 | VS | 21.74 | M |
| 22.84 | VS | 23.59 | M | 24.19 | M |
| 25.56 | W | 26.49 | w | 28.56 | w |
| 29.10 | W | 29.98 | M | 33.12 | w |

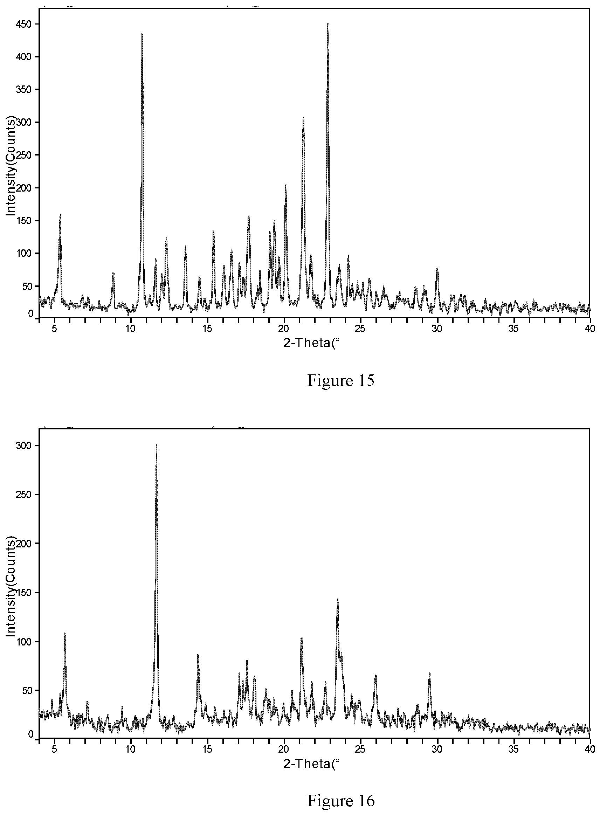

In another preferred embodiment, the X-ray powder diffraction pattern of crystal form H-1 is substantially as characterized in Figure 15.

In another preferred embodiment, the polymorph is H-2 crystalline form of the fumarate of compound of formula X, i.e. crystal form H-2, the X-ray powder diffraction pattern of which has peaks at diffraction angles 2θ(°) values of the following group H-2-1: 5.69±0.2, 11.67±0.2, 14.39±0.2, 21.15±0.2, and 23.49±0.2.

In another preferred embodiment, the X-ray powder diffraction pattern of the crystal form H-2 further has peaks at 2 or more than 2 of diffraction angles 2θ (°) values selected from the following group H-2-2: 17.06±0.2, 17.33±0.2, 17.56±0.2, 18.05±0.2, 18.81±0.2, 21.80±0.2, 22.68±0.2, 23.74±0.2, 25.97±0.2, and 29.48±0.2.

In another preferred embodiment, the X-ray powder diffraction pattern of the crystal form H-2 further has peaks at 2 or more than 2 of diffraction angles 2θ (°) values selected from the following group H-2-3: 7.16±0.2, 9.43±0.2, 14.86±0.2, 19.95±0.2, 20.51±0.2, 24.41±0.2, 24.90±0.2, and 28.70±0.2.

In another preferred embodiment, the X-ray powder diffraction pattern of the crystal form H-2 has peaks at 6 or more or all (such as 6, 7, 8, 9, 10, 11, 12, 13, 14, 15, etc.) of 20 (°) values selected from the groups H-2-1, H-2-2 and H-2-3.

In another preferred embodiment, the X-ray powder diffraction pattern of the crystal form H-2 has peaks at 2θ(°) values shown in table (H-2), and the relative intensity of each peak is as shown in table (H-2):

**Table (H-2)**

| 2θ(°) | I/I₀ | 2θ(°) | I/I₀ | 2θ(°) | I/I₀ |
|---|---|---|---|---|---|
| 5.69 | S | 7.16 | w | 9.43 | W |
| 11.67 | VS | 14.39 | S | 14.86 | W |
| 17.06 | M | 17.33 | M | 17.56 | M |
| 18.05 | M | 18.81 | M | 19.95 | W |
| 20.51 | W | 21.15 | S | 21.80 | M |
| 22.68 | M | 23.49 | S | 23.74 | M |
| 24.41 | W | 24.90 | W | 25.97 | M |
| 28.70 | W | 29.48 | M | | |

In another preferred embodiment, the X-ray powder diffraction pattern of crystal form H-2 is substantially as characterized in Figure 16.

In another preferred embodiment, the polymorph is J crystalline form of the methanesulfonate of compound of formula X, i.e. crystal form J, the X-ray powder diffraction pattern of which has peaks at diffraction angles 2θ(°) values of the following group J1: 10.64±0.2, 18.70±0.2, 20.55±0.2, 20.86±0.2, 21.58±0.2, 22.16±0.2, 23.05±0.2, 24.39±0.2, 24.75±0.2, and 27.18±0.2.

In another preferred embodiment, the X-ray powder diffraction pattern of the crystal form J further has peaks at 2 or more than 2 of diffraction angles 2θ (°) values selected from the following group J2: 4.87±0.2, 8.06±0.2, 9.13±0.2, 9.77±0.2, 12.51±0.2, 13.89±0.2, 14.69±0.2, 15.81±0.2, 16.10±0.2, 17.22±0.2, 17.98±0.2, 19.31±0.2, 19.75±0.2, 20.23±0.2, 23.98±0.2, 25.97±0.2, and 27.43±0.2.

In another preferred embodiment, the X-ray powder diffraction pattern of the crystal form J further has peaks at diffraction angles 2θ (°) values selected from the following group J3: 6.93±0.2 and 16.62±0.2.

In another preferred embodiment, the X-ray powder diffraction pattern of the crystal form J has peaks at 6 or more or all (such as 6, 7, 8, 9, 10, 11, 12, 13, 14, 15, etc.) of 2θ (°) values selected from the groups J1, J2 and J3.

In another preferred embodiment, the X-ray powder diffraction pattern of the crystal form J has peaks at 2θ (°) values shown in table J1, and the relative intensity of each peak is as shown in table J1:

**Table J1**

| 2θ(°) | I/I₀ | 2θ(°) | I/I₀ | 2θ(°) | I/I₀ |
|---|---|---|---|---|---|
| 4.87 | M | 6.93 | W | 8.06 | M |
| 9.13 | M | 9.77 | M | 10.64 | VS |
| 12.51 | M | 13.89 | M | 14.69 | M |
| 15.81 | M | 16.10 | M | 16.62 | W |
| 17.22 | M | 17.98 | M | 18.70 | S |
| 19.31 | M | 19.75 | M | 20.23 | M |
| 20.55 | VS | 20.86 | VS | 21.58 | VS |
| 22.16 | S | 23.05 | S | 23.98 | M |
| 24.39 | S | 24.75 | S | 25.97 | M |
| 27.18 | S | 27.43 | M | | |

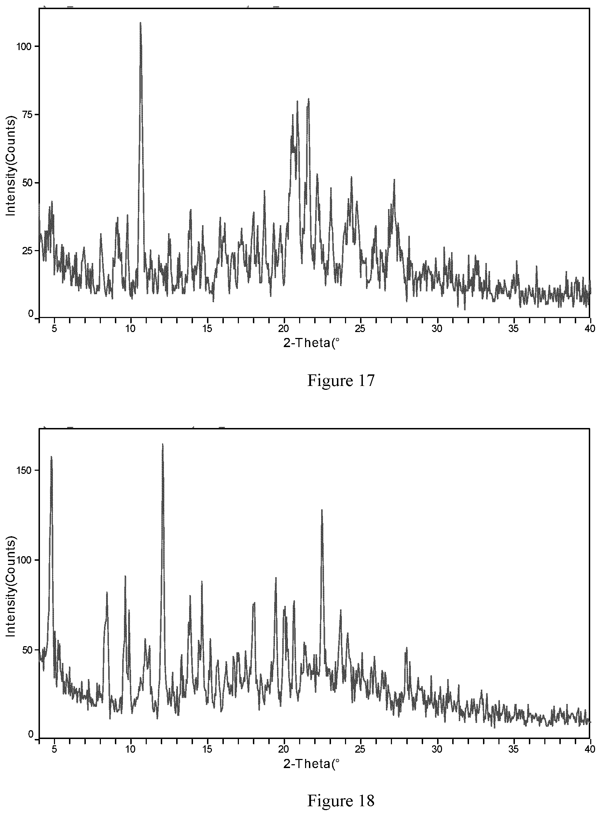

In another preferred embodiment, the X-ray powder diffraction pattern of crystal form J is substantially as characterized in Figure 17.

In another preferred embodiment, the polymorph is K-1 crystalline form of the formate of compound of formula X, i.e. crystal form K-1, the X-ray powder diffraction pattern of which has peaks at diffraction angles 2θ(°) values of the following group K-1-1: 4.80±0.2, 8.43±0.2, 9.63±0.2, 9.88±0.2, 12.08±0.2, 13.87±0.2, 14.63±0.2, 18.02±0.2, 19.44±0.2, 20.05±0.2, 20.64±0.2, 22.47±0.2, and 23.68±0.2.

In another preferred embodiment, the X-ray powder diffraction pattern of the crystal form K-1 further has peaks at 2 or more than 2 of diffraction angles 2θ (°) values selected from the following group K-1-2: 10.98±0.2, 11.21±0.2, 13.32±0.2, 15.17±0.2, 15.65±0.2, 16.96±0.2, 21.33±0.2, 24.15±0.2, 27.96±0.2, and 28.19±0.2.

In another preferred embodiment, the X-ray powder diffraction pattern of the crystal form K-1 further has peaks at 2 or more than 2 of diffraction angles 2θ (°) values selected from the following group K-1-3: 16.19±0.2, 16.70±0.2, and 17.49±0.2.

In another preferred embodiment, the X-ray powder diffraction pattern of the crystal form K-1 has peaks at 6 or more or all (such as 6, 7, 8, 9, 10, 11, 12, 13, 14, 15, etc.) of 2θ (°) values selected from the groups K-1-1, K-1-2 and K-1-3.

In another preferred embodiment, the X-ray powder diffraction pattern of the crystal form K-1 has peaks at 2θ(°) values shown in table (K-1), and the relative intensity of each peak is as shown in table (K-1):

**Table K-1**

| 2θ(°) | I/I₀ | 2θ(°) | I/I₀ | 2θ(°) | I/I₀ |
|---|---|---|---|---|---|
| 4.80 | VS | 8.43 | S | 9.63 | S |
| 9.88 | S | 10.98 | M | 11.21 | M |
| 12.08 | VS | 13.32 | M | 13.87 | S |
| 14.63 | S | 15.17 | M | 15.65 | M |
| 16.19 | W | 16.70 | W | 16.96 | M |
| 17.49 | W | 18.02 | S | 19.44 | S |
| 20.05 | S | 20.64 | S | 21.33 | M |
| 22.47 | VS | 23.68 | S | 24.15 | M |
| 27.96 | M | 28.19 | M | | |

In another preferred embodiment, the X-ray powder diffraction pattern of crystal form K-1 is substantially as characterized in Figure 18.

In another preferred embodiment, the polymorph is K-2 crystalline form of the formate of compound of formula X, i.e. crystal form K-2, the X-ray powder diffraction pattern of which has peaks at diffraction angles 2θ(°) values of the following group K-2-1: 5.23±0.2, 17.05±0.2, 17.31±0.2, 21.28±0.2, and 22.50±0.2.

In another preferred embodiment, the X-ray powder diffraction pattern of the crystal form K-2 further has peaks at 2 or more than 2 of diffraction angles 2θ (°) values selected from the following group K-2-2: 10.53±0.2, 12.33±0.2, 12.75±0.2, 13.99±0.2, 15.54±0.2, 16.44±0.2, 18.23±0.2, 19.47±0.2, and 22.93±0.2.

In another preferred embodiment, the X-ray powder diffraction pattern of the crystal form K-2 further has peaks at diffraction angles 2θ (°) values of the following group K-2-3: 19.86±0.2.

In another preferred embodiment, the X-ray powder diffraction pattern of the crystal form K-2 has peaks at 6 or more or all (such as 6, 7, 8, 9, 10, 11, 12, 13, 14, 15, etc.) of 2θ (°) values selected from the groups K-2-1, K-2-2 and K-2-3.

In another preferred embodiment, the X-ray powder diffraction pattern of the crystal form K-2 has peaks at 2θ(°) values shown in table (K-2), and the relative intensity of each peak is as shown in table (K-2):

**Table (K-2)**

| 2θ(°) | I/I₀ | 2θ(°) | I/I₀ | 2θ(°) | I/I₀ |
|---|---|---|---|---|---|
| 5.23 | VS | 10.53 | M | 12.33 | M |
| 12.75 | M | 13.99 | M | 15.54 | M |
| 16.44 | M | 17.05 | VS | 17.31 | S |
| 18.23 | M | 19.47 | M | 19.86 | W |
| 21.28 | VS | 22.50 | S | 22.93 | M |

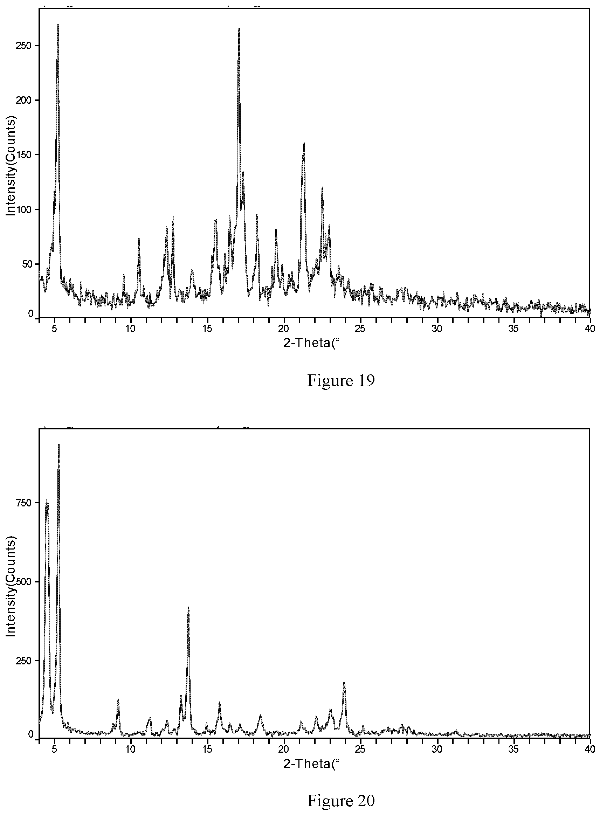

In another preferred embodiment, the X-ray powder diffraction pattern of crystal form K-2 is substantially as characterized in Figure 19.

In another preferred embodiment, the polymorph is K-3 crystalline form of the formate of compound of formula X, i.e. crystal form K-3, the X-ray powder diffraction pattern of which has peaks at diffraction angles 2θ(°) values of the following group K-3-1: 4.51±0.2, 5.27±0.2, and 13.76±0.2.

In another preferred embodiment, the X-ray powder diffraction pattern of the crystal form K-3 further has peaks at diffraction angles 2θ (°) values of the following group K-3-2: 9.17±0.2, 13.28±0.2, 15.77±0.2, and 23.91±0.2.

In another preferred embodiment, the X-ray powder diffraction pattern of the crystal form K-3 further has peaks at 2 or more than 2 of diffraction angles 2θ (°) values selected from the following group K-3-3: 11.25±0.2, 12.36±0.2, 14.93±0.2, 16.46±0.2, 17.11±0.2, 18.45±0.2, 21.11±0.2, 22.09±0.2, and 23.04±0.2.

In another preferred embodiment, the X-ray powder diffraction pattern of the crystal form K-3 has peaks at 6 or more or all (such as 6, 7, 8, 9, 10, 11, 12, 13, 14, 15, etc.) of 2θ (°) values selected from the groups K-3-1, K-3-2 and K-3-3.

In another preferred embodiment, the X-ray powder diffraction pattern of the crystal form K-3 has peaks at 2θ(°) values shown in table (K-3), and the relative intensity of each peak is as shown in table (K-3):

**Table (K-3)**

| 2θ(°) | I/I₀ | 2θ(°) | I/I₀ | 2θ(°) | I/I₀ |
|---|---|---|---|---|---|
| 4.51 | VS | 5.27 | VS | 9.17 | M |
| 11.25 | W | 12.36 | W | 13.28 | M |
| 13.76 | S | 14.93 | W | 15.77 | M |
| 16.46 | W | 17.11 | W | 18.45 | W |
| 21.11 | W | 22.09 | W | 23.04 | W |
| 23.91 | M | | | | |

In another preferred embodiment, the X-ray powder diffraction pattern of crystal form K-3 is substantially as characterized in Figure 20.

In another preferred embodiment, the polymorph is K-4 crystalline form of the formate of compound of formula X, i.e. crystal form K-4, the X-ray powder diffraction pattern of which has peaks at diffraction angles 2θ(°) values of the following group K-4-1: 13.26±0.2, 15.04±0.2, 16.18±0.2, 19.09±0.2, and 21.54±0.2.

In another preferred embodiment, the X-ray powder diffraction pattern of the crystal form K-4 further has peaks at 2 or more than 2 of diffraction angles 2θ (°) values selected from the following group K-4-2: 9.69±0.2, 16.35±0.2, 16.86±0.2, 19.46±0.2, and 20.85±0.2.

In another preferred embodiment, the X-ray powder diffraction pattern of the crystal form K-4 further has peaks at 2 or more than 2 of diffraction angles 2θ (°) values selected from the following group K-4-3: 11.2±0.2, 12.03±0.2, 14.00±0.2, 17.13±0.2, 19.66±0.2, 20.38±0.2, 21.83±0.2, 22.12±0.2, 22.58±0.2, 23.40±0.2, 26.21±0.2, 27.44±0.2, 28.08±0.2, 29.39±0.2, 30.34±0.2, and 33.14±0.2.

In another preferred embodiment, the X-ray powder diffraction pattern of the crystal form K-4 has peaks at 6 or more or all (such as 6, 7, 8, 9, 10, 11, 12, 13, 14, 15, etc.) of 2θ (°) values selected from the groups K-4-1, K-4-2 and K-4-3.

In another preferred embodiment, the X-ray powder diffraction pattern of the crystal form K-4 has peaks at 2θ(°) values shown in table (K-4), and the relative intensity of each peak is as shown in table (K-4):

**Table (K-4)**

| 2θ(°) | I/I₀ | 2θ(°) | I/I₀ | 2θ(°) | I/I₀ |
|---|---|---|---|---|---|
| 9.69 | M | 11.2 | W | 12.03 | W |
| 13.26 | S | 14.00 | W | 15.04 | VS |
| 16.18 | S | 16.35 | M | 16.86 | M |
| 17.13 | W | 19.09 | S | 19.46 | M |
| 19.66 | W | 20.38 | W | 20.85 | M |
| 21.54 | S | 21.83 | W | 22.12 | W |
| 22.58 | W | 23.40 | W | 26.21 | W |
| 27.44 | W | 28.08 | W | 29.39 | W |
| 30.34 | W | 33.14 | W | | |

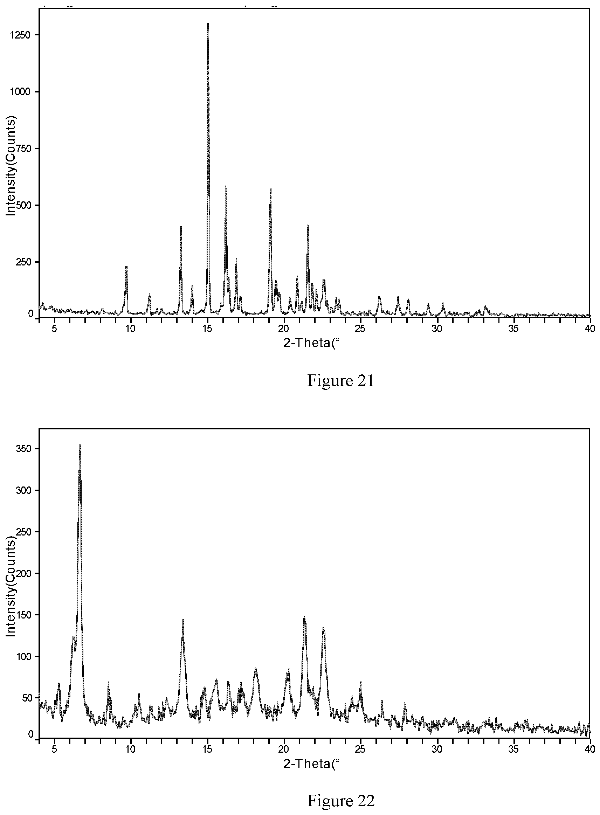

In another preferred embodiment, the X-ray powder diffraction pattern of crystal form K-4 is substantially as characterized in Figure 21.

In another preferred embodiment, the polymorph is crystal form I of the compound of formula X, the X-ray powder diffraction pattern of which has peaks at diffraction angles 2θ(°) values of the group I-1: 6.20±0.2, 6.68±0.2, 13.42±0.2, 21.31±0.2, and 22.56±0.2.

In another preferred embodiment, the X-ray powder diffraction pattern of the crystal form I further has peaks at 2 or more than 2 of diffraction angles 2θ (°) values selected from the following group 1-2: 5.28±0.2, 8.53±0.2, 10.54±0.2, 15.56±0.2, 18.12±0.2, 20.29±0.2, and 24.99±0.2.

In another preferred embodiment, the X-ray powder diffraction pattern of the crystal form I further has peaks at 2 or more than 2 of diffraction angles 2θ (°) values selected from the following group 1-3: 12.34±0.2, 14.80±0.2, 16.35±0.2, 17.17±0.2, 26.40±0.2, and 27.86±0.2.

In another preferred embodiment, the X-ray powder diffraction pattern of the crystal form I has peaks at 6 or more or all (such as 6, 7, 8, 9, 10, 11, 12, 13, 14, 15, etc.) of 2θ (°) values selected from the groups 1-1,1-2 and 1-3.

In another preferred embodiment, the X-ray powder diffraction pattern of the crystal form I has peaks at 2θ(°) values shown in table I1, and the relative intensity of each peak is as shown in table I1:

**Table I1**

| 2θ(°) | I/I₀ | 2θ(°) | I/I₀ | 2θ(°) | I/I₀ |
|---|---|---|---|---|---|
| 5.28 | M | 6.20 | S | 6.68 | VS |
| 8.53 | M | 10.54 | M | 12.34 | W |
| 13.42 | S | 14.80 | W | 15.56 | M |
| 16.35 | W | 17.17 | W | 18.12 | M |
| 20.29 | M | 21.31 | S | 22.56 | S |
| 24.99 | M | 26.40 | W | 27.86 | W |

In another preferred embodiment, the X-ray powder diffraction pattern of crystal form I is substantially as characterized in Figure 22.

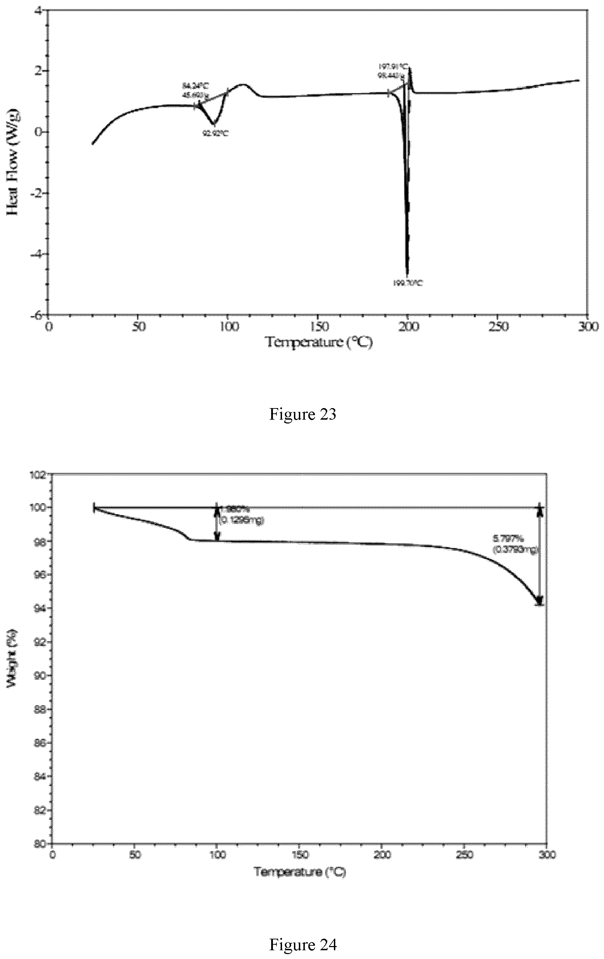

In another preferred embodiment, the crystal form I further has one or more features selected from the following group:
(i) In the differential scanning calorimetry analysis spectrum, the initial temperature are 80±2 °C and 189±2°C; preferably, the differential scanning calorimetry analysis spectrum is substantially as characterized in Figure 23;
(ii) the thermogravimetric analysis spectrum is as characterized in Figure 24;
(iii) The melting point of the crystal form I is in a range of 189-197 °C, preferably 199-201 °C.

In another preferred embodiment, the polymorph is crystal form II of the compound of formula X, the X-ray powder diffraction pattern of which has peaks at diffraction angles 2θ(°) values of the group II-1: 13.27±0.2, 15.03±0.2, 16.20±0.2, 19.09±0.2, and 21.56±0.2.

In another preferred embodiment, the X-ray powder diffraction pattern of the crystal form II further has peaks at 2 or more than 2 of diffraction angles 2θ (°) values selected from the following group II-2: 9.71±0.2, 11.21±0.2, 14.01±0.2, 16.35±0.2, 19.46±0.2, 19.69±0.2, 20.86±0.2, 22.56±0.2, and 26.24±0.2.

In another preferred embodiment, the X-ray powder diffraction pattern of the crystal form II further has peaks at 2 or more than 2 of diffraction angles 2θ (°) values selected from the following group II-3: 11.98±0.2, 15.89±0.2, 16.86±0.2, 20.39±0.2, 21.15±0.2, 21.83±0.2, 23.09±0.2, 27.43±0.2, 28.08±0.2, and 29.43±0.2.

In another preferred embodiment, the X-ray powder diffraction pattern of the crystal form II has peaks at 6 or more or all (such as 6, 7, 8, 9, 10, 11, 12, 13, 14, 15, etc.) of 2θ (°) values selected from the groups II-1, II-2 and II-3.

In another preferred embodiment, the X-ray powder diffraction pattern of the crystal form II has peaks at 2θ(°) values shown in table II1, and the relative intensity of each peak is as shown in table III:

**Table III**

| 2θ(°) | I/I₀ | 2θ(°) | I/I₀ | 2θ(°) | I/I₀ |
|---|---|---|---|---|---|
| 9.71 | M | 11.21 | M | 11.98 | W |
| 13.27 | S | 14.01 | M | 15.03 | VS |
| 15.89 | W | 16.20 | S | 16.35 | M |
| 16.86 | W | 19.09 | S | 19.46 | M |
| 19.69 | M | 20.39 | W | 20.86 | M |
| 21.15 | W | 21.56 | S | 21.83 | W |
| 22.56 | M | 23.09 | W | 26.24 | M |
| 27.43 | W | 28.08 | W | 29.43 | W |

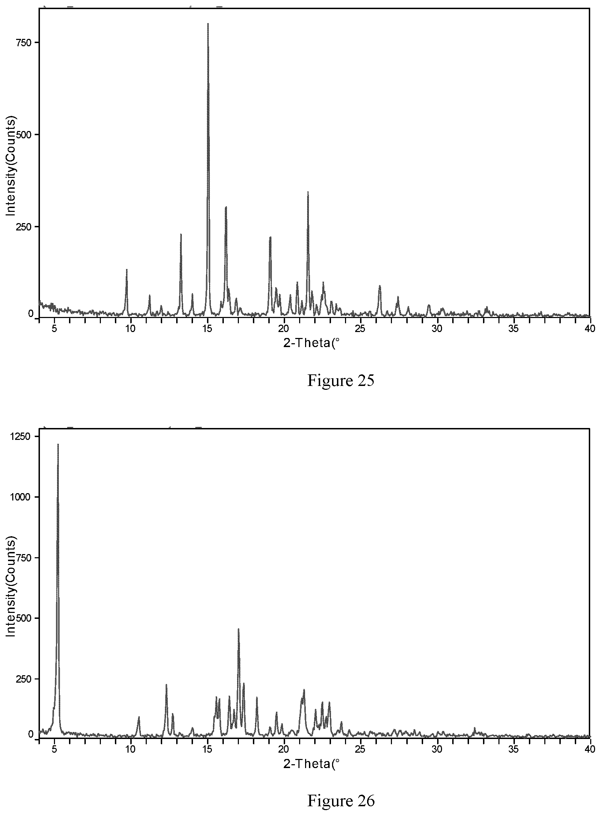

In another preferred embodiment, the X-ray powder diffraction pattern of crystal form II is substantially as characterized in Figure 25.

In another preferred embodiment, the polymorph is crystal form III of the compound of formula X, the X-ray powder diffraction pattern of which has peaks at diffraction angles 2θ(°) values of the group III-1: 5.23±0.2, and 17.02±0.2.

In another preferred embodiment, the X-ray powder diffraction pattern of the crystal form III further has peaks at diffraction angles 2θ (°) values of the group III-2: 12.32±0.2, 12.74±0.2, 15.57±0.2, 16.41±0.2, 16.72±0.2, 17.34±0.2, 18.21±0.2, 19.50±0.2, 21.29±0.2, 22.06±0.2, 22.49±0.2, and 22.94±0.2.

In another preferred embodiment, the X-ray powder diffraction pattern of the crystal form III further has peaks at 2 or more than 2 of diffraction angles 2θ (°) values selected from the following group III-3: 10.51±0.2, 14.01±0.2, 19.07±0.2, 19.84±0.2, 23.73±0.2, 24.25±0.2, and 28.49±0.2.

In another preferred embodiment, the X-ray powder diffraction pattern of the crystal form III has peaks at 6 or more or all (such as 6, 7, 8, 9, 10, 11, 12, 13, 14, 15, etc.) of 2θ (°) values selected from the groups III-1, III-2 and III-3.

In another preferred embodiment, the X-ray powder diffraction pattern of the crystal form III has peaks at 2θ(°) values shown in table III1, and the relative intensity of each peak is as shown in table III1:

**Table III1**

| 2θ(°) | I/I₀ | 2(°) | I/I₀ | 2θ(°) | I/I₀ |
|---|---|---|---|---|---|
| 5.23 | VS | 10.51 | W | 12.32 | M |
| 12.74 | M | 14.01 | W | 15.57 | M |
| 16.41 | M | 16.72 | M | 17.02 | S |
| 17.34 | M | 18.21 | M | 19.07 | W |
| 19.50 | M | 19.84 | W | 21.29 | M |
| 22.06 | M | 22.49 | M | 22.94 | M |
| 23.73 | W | 24.25 | W | 28.49 | W |

In another preferred embodiment, the X-ray powder diffraction pattern of crystal form III is substantially as characterized in Figure 26.

In another preferred embodiment, the polymorph is crystal form IV of the compound of formula X, the X-ray powder diffraction pattern of which has peaks at diffraction angles 2θ(°) values of the group IV-1: 5.25±0.2, 11.94±0.2, 12.23±0.2, 14.42±0.2, and 16.65±0.2.

In another preferred embodiment, the X-ray powder diffraction pattern of the crystal form IV further has peaks at 2 or more than 2 of diffraction angles 2θ (°) values selected from the following group IV-2: 5.60±0.2, 15.97±0.2, 17.45±0.2, 17.96±0.2, 18.81±0.2, 19.35±0.2, 20.58±0.2, 21.00±0.2, and 22.17±0.2.

In another preferred embodiment, the X-ray powder diffraction pattern of the crystal form IV further has peaks at 2 or more than 2 of diffraction angles 2θ (°) values selected from the following group IV-3: 8.13±0.2, 11.25±0.2, 13.17±0.2, 17.02±0.2, 22.78±0.2, and 23.94±0.2.

In another preferred embodiment, the X-ray powder diffraction pattern of the crystal form IV has peaks at 6 or more or all (such as 6, 7, 8, 9, 10, 11, 12, 13, 14, 15, etc.) of 2θ (°) values selected from the groups IV-1, IV-2 and IV-3.

In another preferred embodiment, the X-ray powder diffraction pattern of the crystal form IV has peaks at 2θ(°) values shown in table IV1, and the relative intensity of each peak is as shown in table IV1:

**Table IV1**

| 2θ(°) | I/I₀ | 2θ(°) | I/I₀ | 2θ(°) | I/I₀ |
|---|---|---|---|---|---|
| 5.25 | VS | 5.60 | M | 8.13 | W |
| 11.25 | W | 11.94 | S | 12.23 | S |
| 13.17 | W | 14.42 | S | 15.97 | M |
| 16.65 | S | 17.02 | W | 17.45 | M |
| 17.96 | M | 18.81 | M | 19.35 | M |
| 20.58 | M | 21.00 | M | 22.17 | M |
| 22.78 | W | 23.94 | W | | |

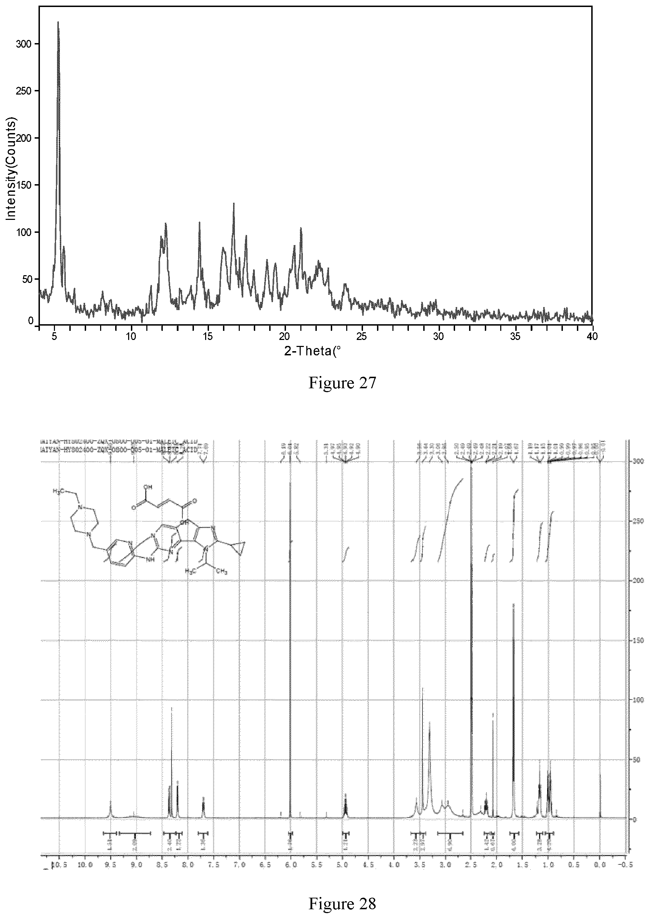

In another preferred embodiment, the X-ray powder diffraction pattern of crystal form IV is substantially as characterized in Figure 27.

In a second aspect of the disclosure, there is provided a process for preparing the pharmaceutically acceptable salt of the compound of formula X, or the polymorph of the compound of formula X or its pharmaceutically acceptable salt according to the first aspect of the present disclosure, comprising steps:
(1) compound 2a is reacted with compound 3a in a solvent thereby to form the compound of formula X; and 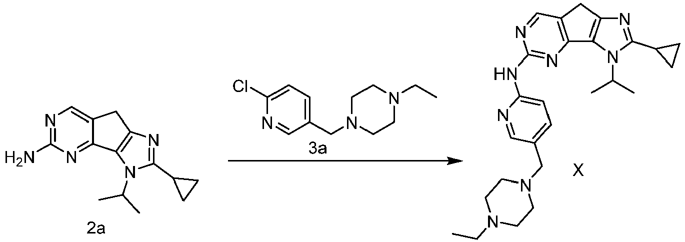
(2) optionally, the compound of formula X and an acid conduct a salt-forming reaction thereby to form a pharmaceutically acceptable salt;
(3) the compound of formula X formed in step (1), or a pharmaceutically acceptable salt thereof formed in step (2) is optionally crystallized thereby to obtain a polymorph.

In another preferred embodiment, in step (2), the solvent is selected from the group consisting of acetonitrile, 50% acetonitrile/50% water, methanol, ethanol, isopropanol, acetone, ethyl acetate, methyl tert-butyl ether, tetrahydrofuran, n-heptane, and dimethyl sulfoxide, preferably acetone, ethanol, ethyl acetate, or 50% acetonitrile/50% water.

In another preferred embodiment, the process comprises any one of the following sub-processes:
(A)the polymorph is A crystalline form of the maleate of compound of formula X, i.e. crystal form A, and the step (3) comprises crystallization process of the compound of formula X in a solvent in the presence of maleic acid, thereby forming the crystal form A.

In another preferred embodiment, in step (A), the solvent is selected from the group consisting of water, 50% acetone/50% water, acetone, acetonitrile, ethyl acetate, ethanol, isopropanol, 50% acetonitrile/50% water, methanol and tetrahydrofuran, preferably, the organic solvent is acetone, ethanol, ethyl acetate, 50% acetonitrile/50% water or 50% acetone/50% water, acetonitrile, isopropanol, or tetrahydrofuran.

In another preferred embodiment, in step (A), the molar ratio of maleic acid to the compound of formula X is (1∼2): 1, preferably (1.0∼1.2): 1.

In another preferred embodiment, in step (A), the crystallization process is slow volatilization, slow cooling or suspension shaking.

In another preferred embodiment, in step (A), the temperature for crystallization process is in a range of 0-100°C, preferably 25-80°C.

In another preferred embodiment, in step (A), the time for crystallization process is 0.5-24 hours.

(B-1) the polymorph is B-1 crystalline form of the hydrochloride of compound of formula X, i.e. crystal form B-1, and the step (3) comprises crystallization process of the compound of formula X in a solvent in the presence of hydrochloric acid, thereby forming the crystal form B-1.

In another preferred embodiment, in step (B-1), the solvent is selected from the group consisting of water, 50% acetone/50% water, acetone, acetonitrile, ethyl acetate, ethanol, isopropanol, 50% acetonitrile/50% water, methanol and tetrahydrofuran, preferably, the organic solvent is acetone, ethanol, methanol, ethyl acetate, acetonitrile, isopropanol, or tetrahydrofuran.

In another preferred embodiment, in step (B-1), the molar ratio of hydrochloric acid to the compound of formula X is (1-2): 1, preferably (1.0∼1.2): 1.

In another preferred embodiment, in step (B-1), the crystallization process is performed by slow volatilization, slow cooling or suspension shaking.

In another preferred embodiment, in step (B-1), the temperature for crystallization process is in a range of 0-100°C, preferably 25-80°C.

In another preferred embodiment, in step (B-1), the time for crystallization process is 0.5-24 hours.

(B-2) the polymorph is B-2 crystalline form of the hydrochloride of compound of formula X, i.e. crystal form B-2, and the step (3) comprises crystallization process of crystal form B-1 in a solvent, thereby forming the crystal form B-2.

In another preferred embodiment, in step (B-2), the solvent is acetonitrile.

In another preferred embodiment, in step (B-2), the molar ratio of hydrochloric acid to the compound of formula X is (1∼2): 1, preferably (1.0∼1.2): 1.

In another preferred embodiment, in step (B-2), the crystallization process is slow cooling.

In another preferred embodiment, in step (B-2), the temperature for crystallization process is in a range of 0-60°C.

In another preferred embodiment, in step (B-2), the time for crystallization process is in a range of 2-3 days.

(B-3) the polymorph is B-3 crystalline form of the hydrochloride of compound of formula X, i.e. crystal form B-3, and the step (3) comprises crystallization process of crystal form B-1 in a solvent, thereby forming the crystal form B-3.

In another preferred embodiment, in step (B-3), the solvent is acetone.

In another preferred embodiment, in step (B-3), the molar ratio of hydrochloric acid to the compound of formula X is (1∼2): 1, preferably (1.0∼1.2): 1.

In another preferred embodiment, in step (B-3), the crystallization process is slow cooling.

In another preferred embodiment, in step (B-3), the temperature for crystallization process is in a range of 0-60°C.

In another preferred embodiment, in step (B-3), the time for crystallization process is in a range of 2-3 days.

(C-1) the polymorph is C-1 crystalline form of the sulfate of compound of formula X, i.e. crystal form C-1, and the step (3) comprises crystallization process of the compound of formula X in a solvent in the presence of sulfuric acid, thereby forming the crystal form C-1.

In another preferred embodiment, in step (C-1), the solvent is ethanol or acetone.

In another preferred embodiment, in step (C-1), the crystallization process is slow volatilization.

In another preferred embodiment, in step (C-1), the temperature for crystallization process is in a range of 0-60°C, preferably 4-50°C.

In another preferred embodiment, in step (C-1), the time for crystallization process is in a range of 1 hour-3 days.

(C-2) the polymorph is C-2 crystalline form of the sulfate of compound of formula X, i.e. crystal form C-2, and the step (3) comprises crystallization process of the compound of formula X in a solvent in the presence of sulfuric acid, thereby forming the crystal form C-2.

In another preferred embodiment, in step (C-2), the solvent is ethyl acetate.

In another preferred embodiment, in step (C-2), the crystallization process is slow volatilization.

In another preferred embodiment, in step (C-2), the temperature for crystallization process is in a range of 0-60°C, preferably 4-50°C.

In another preferred embodiment, in step (C-2), the time for crystallization process is in a range of 1 hour-3 days.

(D) the polymorph is D crystalline form of the hydrobromide of compound of formula X, i.e. crystal form D, and the step (3) comprises crystallization process of the compound of formula X in a solvent in the presence of hydrobromic acid, thereby forming the crystal form D.

In another preferred embodiment, in step (D), the solvent is ethyl acetate.

In another preferred embodiment, in step (D), the crystallization process is slow volatilization.

In another preferred embodiment, in step (D), the temperature for crystallization process is in a range of 0-60°C, preferably 4-50°C.

In another preferred embodiment, in step (D), the time for crystallization process is in a range of 1 hour-3 days.

(E) the polymorph is E crystalline form of the L-tartrate of compound of formula X, i.e. crystal form E, and the step (3) comprises crystallization process of the compound of formula X in a solvent in the presence of L-tartaric acid, thereby forming the crystal form E.

In another preferred embodiment, in step (E), the solvent is acetone.

In another preferred embodiment, in step (E), the crystallization process is slow volatilization.

In another preferred embodiment, in step (E), the temperature for crystallization process is in a range of 0-60°C, preferably 4-50°C.

In another preferred embodiment, in step (E), the time for crystallization process is in a range of 1 hour-3 days.

(F) the polymorph is F crystalline form of the phosphate of compound of formula X, i.e. crystal form F, and the step (3) comprises crystallization process of the compound of formula X in a solvent in the presence of phosphoric acid, thereby forming the crystal form F.

In another preferred embodiment, in step (F), the solvent is acetone.

In another preferred embodiment, in step (F), the crystallization process is slow volatilization.

In another preferred embodiment, in step (F), the temperature for crystallization process is in a range of 0-60°C, preferably 4-50°C.

In another preferred embodiment, in step (F), the time for crystallization process is in a range of 1 hour-3 days.

(G) the polymorph is G crystalline form of the citrate of compound of formula X, i.e. crystal form G, and the step (3) comprises crystallization process of the compound of formula X in a solvent in the presence of citric acid, thereby forming the crystal form G.

In another preferred embodiment, in step (G), the solvent is acetone.

In another preferred embodiment, in step (G), the crystallization process is slow volatilization.

In another preferred embodiment, in step (G), the temperature for crystallization process is in a range of 0-60°C, preferably 4-50°C.

In another preferred embodiment, in step (G), the time for crystallization process is in a range of 1 hour-3 days.

(H-1) the polymorph is H-1 crystalline form of the fumarate of compound of formula X, i.e. crystal form H-1, and the step (3) comprises crystallization process of the compound of formula X in a solvent in the presence of fumaric acid, thereby forming the crystal form H-1.

In another preferred embodiment, in step (H-1), the solvent is acetone.

In another preferred embodiment, in step (H-1), the crystallization process is slow volatilization.

In another preferred embodiment, in step (H-1), the temperature for crystallization process is in a range of 0-60°C, preferably 4-50°C.

In another preferred embodiment, in step (H-1), the time for crystallization process is in a range of 1 hour-3 days.

(H-2) the polymorph is H-2 crystalline form of the fumarate of compound of formula X, i.e. crystal form H-2, and the step (3) comprises crystallization process of the compound of formula X in a solvent in the presence of fumaric acid, thereby forming the crystal form H-2.

In another preferred embodiment, in step (H-2), the solvent is ethanol or ethyl acetate.

In another preferred embodiment, in step (H-2), the crystallization process is slow volatilization.

In another preferred embodiment, in step (H-2), the temperature for crystallization process is in a range of 0-60°C, preferably 4-50°C.

In another preferred embodiment, in step (H-2), the time for crystallization process is in a range of 1 hour-3 days.

(J) the polymorph is J crystalline form of the methanesulfonate of compound of formula X, i.e. crystal form J, and the step (3) comprises crystallization process of the compound of formula X in a solvent in the presence of methanesulfonic acid, thereby forming the crystal form J.

In another preferred embodiment, in step (J), the solvent is ethanol, acetone, 50% acetonitrile/50% water, or ethyl acetate.

In another preferred embodiment, in step (J), the crystallization process is slow volatilization.

In another preferred embodiment, in step (J), the temperature for crystallization process is in a range of 0-60°C, preferably 4-50°C.

In another preferred embodiment, in step (J), the time for crystallization process is in a range of 1 hour-3 days.

(K-1) the polymorph is K-1 crystalline form of the formate of compound of formula X, i.e. crystal form K-1, and the step (3) comprises crystallization process of the compound of formula X in a solvent in the presence of formic acid, thereby forming the crystal form K-1.

In another preferred embodiment, in step (K-1), the solvent is ethyl acetate, tetrahydrofuran, methanol, isopropanol or ethanol.

In another preferred embodiment, in step (K-1), the crystallization process is slow volatilization.

In another preferred embodiment, in step (K-1), the temperature for crystallization process is in a range of 0-80°C.

In another preferred embodiment, in step (K-1), the time for crystallization process is in a range of 1 hour-5 days.

(K-2) the polymorph is K-2 crystalline form of the formate of compound of formula X, i.e. crystal form K-2, and the step (3) comprises crystallization process of crystal form K-1 in a solvent, thereby forming the crystal form K-2.

In another preferred embodiment, in step (K-2), the solvent is acetonitrile or acetone.

In another preferred embodiment, in step (K-2), the crystallization process is slow volatilization.

In another preferred embodiment, in step (K-2), the temperature for crystallization process is in a range of 0-80°C.

In another preferred embodiment, in step (K-2), the time for crystallization process is in a range of 1 hour-5 days.

(K-3) the polymorph is K-3 crystalline form of the formate of compound of formula X, i.e. crystal form K-3, and the step (3) comprises crystallization process of crystal form K-1 in a solvent, thereby forming the crystal form K-3.

In another preferred embodiment, in step (K-3), the solvent is acetonitrile.

In another preferred embodiment, in step (K-3), the crystallization process is slow cooling.

In another preferred embodiment, in step (K-3), the temperature for crystallization process is in a range of 0-80°C.

In another preferred embodiment, in step (K-3), the time for crystallization process is in a range of 1 hour-5 days.

(K-4) the polymorph is K-4 crystalline form of the formate of compound of formula X, i.e. crystal form K-4, and the step (3) comprises crystallization process of crystal form K-1 in a solvent, thereby forming the crystal form K-4.

In another preferred embodiment, in step (K-4), the solvent is acetone.

In another preferred embodiment, in step (K-4), the crystallization process is slow cooling.

In another preferred embodiment, in step (K-4), the temperature for crystallization process is in a range of 0-80°C.

In another preferred embodiment, in step (K-4), the time for crystallization process is in a range of 1 hour-5 days.

(I) the polymorph is crystal form I of the compound of formula X, and the step (3) comprises crystallization process of the compound of formula X in a solvent, thereby forming the crystal form I.

In another preferred embodiment, in step (I), the solvent is selected from the group consisting of water, methanol, ethanol, propanol, isopropanol, butanol, acetone, acetonitrile, tetrahydrofuran, propylene glycol, ethyl acetate, methyl isobutyl ketone, isopropyl acetate, 2-methyltetrahydrofuran, dichloromethane, methyl tert-butyl ether, dimethyl sulfoxide, toluene, N,N-dimethylacetamide, N-methylpyrrolidone, or a combination thereof.

In another preferred embodiment, in step (I), the solvent is selected from the group consisting of water, acetonitrile, 50% acetonitrile/50% water, methanol, ethanol, isopropanol, acetone, ethyl acetate, methyl tert-butyl ether, tetrahydrofuran, n-heptane, and dimethyl sulfoxide.

In another preferred embodiment, in step (I), the crystallization process is slow volatilization.

In another preferred embodiment, in step (I), 5-30°C, preferably 10-20°C.

In another preferred embodiment, in step (I), the time for crystallization process is in a range of 1-10 days, preferably 4-8 days.

(II) the polymorph is crystal form II of the compound of formula X, and the step (3) comprises crystallization process of the compound of formula X in a solvent, thereby forming the crystal form II.

In another preferred embodiment, in step (II), the solvent is selected from the group consisting of 50% acetonitrile/50% water, water, methanol, ethanol, propanol, isopropanol, butanol, acetone, acetonitrile, tetrahydrofuran, propylene glycol, ethyl acetate, methyl isobutyl ketone, isopropyl acetate, 2-methyltetrahydrofuran, dichloromethane, methyl tert-butyl ether, dimethyl sulfoxide, toluene, N,N-dimethylacetamide, N-methylpyrrolidone, or a combination thereof. Isopropanol, ethanol, ethyl acetate, acetone, acetonitrile, or 50%acetonitrile/50%water is preferred.

In another preferred embodiment, in step (II), the crystallization process is slow volatilization.

In another preferred embodiment, in step (II), 0-60°C, preferably 5-40°C.

In another preferred embodiment, in step (II), the time for crystallization process is in a range of 0.5 hour-10 days.

(III) the polymorph is crystal form III of the compound of formula X, and the step (3) comprises crystallization process of the compound of formula X in a solvent, thereby forming the crystal form III.

In another preferred embodiment, in step (III), the solvent is selected from the group consisting of water, n-heptane, methanol, ethanol, propanol, isopropanol, butanol, acetone, acetonitrile, tetrahydrofuran, propylene glycol, ethyl acetate, methyl isobutyl ketone, isopropyl acetate, 2-methyltetrahydrofuran, dichloromethane, methyl tert-butyl ether, dimethyl sulfoxide, toluene, N,N-dimethylacetamide, N-methylpyrrolidone, or a combination thereof.Methyl tert-butyl ether or n-heptane is preferred.

In another preferred embodiment, in step (III), the crystallization process is slow volatilization.

In another preferred embodiment, in step (III), 0-60°C, preferably 5-40°C.

In another preferred embodiment, in step (III), the time for crystallization process is in a range of 0.5 hour-10 days.

(IV) the polymorph is crystal form IV of the compound of formula X, and the step (3) comprises crystallization process of the compound of formula X in a solvent, thereby forming the crystal form IV

In another preferred embodiment, in step (IV), the solvent is selected from the group consisting of water, methanol, ethanol, propanol, isopropanol, butanol, acetone, acetonitrile, tetrahydrofuran, propylene glycol, ethyl acetate, methyl isobutyl ketone, isopropyl acetate, 2-methyltetrahydrofuran, dichloromethane, methyl tert-butyl ether, dimethyl sulfoxide, toluene, N,N-dimethylacetamide, N-methylpyrrolidone, or a combination thereof. Tetrahydrofuran is preferred.

In another preferred embodiment, in step (IV), the crystallization process is slow volatilization.

In another preferred embodiment, in step (IV), 0-60°C, preferably 5-40°C.

In another preferred embodiment, in step (IV), the time for crystallization process is in a range of 0.5 hour-10 days.

In a third aspect of the disclosure, there is provided a pharmaceutical composition comprising:
(a) the pharmaceutically acceptable salt of the compound of formula X, the polymorph of the compound of formula X, or the polymorph of pharmaceutically acceptable salt of the compound of formula X according to any one of the first aspect of the present disclosure; and (b) a pharmaceutically acceptable carrier.

In a fourth aspect of the disclosure, there is provided a use of the pharmaceutically acceptable salt of the compound of formula X, the polymorph of the compound of formula X, or the polymorph of pharmaceutically acceptable salt of the compound of formula X according to the first aspect of the present disclosure, or the pharmaceutical composition according to the third aspect of the present disclosure in the preparation of a drug for the treatment of diseases or disorders, the disease or disorder is selected from the group consisting of cancer, abnormal cell proliferative diseases, infections, inflammatory disorders, autoimmune diseases, cardiovascular diseases, neurodegenerative diseases, radiation-induced hematopoietic toxic diseases, or a combination thereof.

In another preferred embodiment, the cancer is selected from the group consisting of breast cancer, ovarian cancer, prostate cancer, melanoma, brain tumor, esophageal cancer, stomach cancer, liver cancer, pancreatic cancer, colorectal cancer, lung cancer, kidney cancer, skin cancer, glioblastoma, neuroblastoma, sarcoma, liposarcoma, osteochondroma, osteoma, osteosarcoma, seminoma, testicular tumor, uterine cancer, head and neck tumor, multiple myeloma, malignant lymphoma, polycythemia vera, leukemia, thyroid tumor, ureteral tumor, bladder tumor, gallbladder cancer, cholangiocarcinoma, chorionic epithelioma and pediatric tumor.

In another preferred embodiment, the radiation-induced hematopoietic toxic diseases include, but are not limited to, myelosuppression, neutropenia, leukopenia, and anemia.

In a fifth aspect of the present disclosure, there is provided a method of inhibiting CDK4 and/or CDK6 activity, comprising administering to a patient in need thereof a therapeutically effective amount of the pharmaceutically acceptable salt of the compound of formula X, the polymorph of the compound of formula X, or the polymorph of pharmaceutically acceptable salt of the compound of formula X according to the first aspect of the present disclosure, or the pharmaceutical composition according to the third aspect of the present disclosure.

In a sixth aspect of the present disclosure, there is provided a method of treating abnormal cell proliferative diseases, infections (for example, viral infections such as herpes, HIV, fungal infections, etc.), inflammatory diseases (for example, rheumatoid arthritis, osteoarthritis, etc.), and autoimmune disease (such as psoriasis, lupus, type I diabetes, diabetic nephropathy, multiple sclerosis, glomerulonephritis, etc.), cardiovascular diseases (such as myocardial infarction, stroke, atherosclerosis, postoperative vascular stenosis, restenosis, etc.) or neurodegenerative diseases (such as Alzheimer's disease, Parkinson's disease, etc.), comprising administering to a patient in need thereof a therapeutically effective amount of the pharmaceutically acceptable salt of the compound of formula X, the polymorph of the compound of formula X or the polymorph of pharmaceutically acceptable salt of the compound of formula X according to the first aspect of the present disclosure, or the pharmaceutical composition according to the third aspect of the present disclosure, wherein the abnormal cell proliferative disease may be cancer.

In a seventh aspect of the present disclosure, there is provided a method of treating cancer, comprising administering to a patient in need thereof a therapeutically effective amount of the pharmaceutically acceptable salt of the compound of formula X, the polymorph of the compound of formula X, or the polymorph of pharmaceutically acceptable salt of the compound of formula X according to the first aspect of the present disclosure, or the pharmaceutical composition according to the third aspect of the present disclosure, wherein the cancer is selected from the group consisting of breast cancer, ovarian cancer, prostate cancer, melanoma, and brain tumor (e.g. gliomas with malignant astroglial and oligodendroglioma components, etc.), esophageal cancer, stomach cancer, liver cancer, pancreatic cancer, colorectal cancer (eg, colon cancer, rectal cancer, etc.), lung cancer (eg, non-small cell lung cancer, small cell lung cancer, primary or metastatic squamous carcinoma, etc.), kidney cancer, skin cancer, glioblastoma, neuroblastoma, sarcoma, liposarcoma, osteochondroma, osteoma, osteosarcoma, seminoma, testicular tumor, uterine cancer (for example, cervical cancer, endometrial cancer, etc.), head and neck tumor (such as maxillary cancer, laryngeal cancer, pharynx cancer, tongue cancer, intraoral cancer, etc.), multiple myeloma, malignant lymphoma (such as reticulocyte sarcoma, lymphoid sarcoma, Hodgkin's lymphoma, mantle cell lymphoma, etc.), polycythemia vera, leukemia (eg, acute granulocytic leukemia, chronic granulocytic leukemia, acute lymphoblastic leukemia, chronic lymphocytic leukemia, etc.), thyroid tumor, ureteral tumor, bladder tumor, gallbladder cancer, cholangiocarcinoma, chorionic epithelioma and paediatric tumor (eg Ewing familial sarcoma, Wilms sarcoma, rhabdomyosarcoma, hemangiosarcoma, embryonic testicular cancer, neuroblastoma, retinoblastoma, hepatoblastoma, nephroblastoma, etc.).

It should be understood that each of the above technical features of the disclosure and each technical feature specifically described below (such as in Examples) can be combined with each other within the scope of the present disclosure so as to constitute new or preferred technical solutions which need not be specified again herein.

### BRIEF DESCRIPTION OF THE DRAWINGS

Figure 1 is the X-ray powder diffraction pattern of the crystal form A.
Figure 2 is the differential scanning calorimetry analysis spectrum of the crystal form A.
Figure 3 is the thermogravimetric analysis spectrum of the crystal form A.
Figure 4 is the X-ray powder diffraction pattern of the crystal form B-1.
Figure 5 is the the differential scanning calorimetry analysis spectrum of the crystal form B-1.
Figure 6 is the thermogravimetric analysis spectrum of the crystal form B-1.
Figure 7 is the X-ray powder diffraction pattern of the crystal form B-2.
Figure 8 is the X-ray powder diffraction pattern of the crystal form B-3.
Figure 9 is the X-ray powder diffraction pattern of the crystal form C-1.
Figure 10 is the X-ray powder diffraction pattern of the crystal form C-2.
Figure 11 is the X-ray powder diffraction pattern of the crystal form D.
Figure 12 is the X-ray powder diffraction pattern of the crystal form E.
Figure 13 is the X-ray powder diffraction pattern of the crystal form F.
Figure 14 is the X-ray powder diffraction pattern of the crystal form G.
Figure 15 is the X-ray powder diffraction pattern of the crystal form H-1.
Figure 16 is the X-ray powder diffraction pattern of the crystal form H-2.
Figure 17 is the X-ray powder diffraction pattern of the crystal form J.
Figure 18 is the X-ray powder diffraction pattern of the crystal form K-1.
Figure 19 is the X-ray powder diffraction pattern of the crystal form K-2.
Figure 20 is the X-ray powder diffraction pattern of the crystal form K-3.
Figure 21 is the X-ray powder diffraction pattern of the crystal form K-4.
Figure 22 is the X-ray powder diffraction pattern of the crystal form I.
Figure 23 is the differential scanning calorimetry analysis spectrum of the crystal form I.
Figure 24 is the thermogravimetric analysis spectrum of the crystal form I.
Figure 25 is the X-ray powder diffraction pattern of the crystal form II.
Figure 26 is the X-ray powder diffraction pattern of the crystal form III.
Figure 27 is the X-ray powder diffraction pattern of the crystal form IV
Figure 28 is the ¹HNMR spectrum of the crystal form A.
Figure 29 is the XRD pattern of crystal form B-1 under accelerated conditions for 0 days-28 days.
Figure 30 is the XRD pattern of crystal form A under accelerated conditions for 0 days-28 days.
Figure 31 is the XRD pattern of crystal form A at 60 °C for 14 days.
Figure 32 is the XRD pattern of crystal form A at 60 °C for 32 days.
Figure 33 is the DVS pattern of the crystal form I.
Figure 34 is the DVS pattern of the crystal form A.
Figure 35 is the DVS pattern of the crystal form B-1.
Figure 36 is the DVS pattern of the crystal form K-1.

### DETAIL DESCRIPTION OF INVENTION

After extensive and in-depth research, the present inventors unexpectedly discovered a series of polymorphs of free base, salts and polymorphs of the salts of the compound of formula X, which have a high inhibitory activity against CDK4 and CDK6, and weak inhibitory activity against CDK1 and CDK2, with obvious 4, 6 selectivity. The study also found that a series of polymorphs of free base, salts and the polymorphs of the salts of the compound of formula X not only had good physical and chemical stability, but also had good pharmacological activity *in vivo* and *in vitro,* and therefore could be further developed into drugs.

### TERMS

As used herein, "the crystal of the present disclosure", "the crystal form of the present disclosure", "the polymorph of the present disclosure", etc. can be used interchangeably.

### Compound of formula X

In the present disclosure, the compound of formula X is 2-cyclopropyl-N-(5-((4-ethylpiperazin-1-yl) methyl)pyridin-2-yl)-3-isopropyl-3,8-dihydroimidazo[4',5',4,5]cyclopenta[1,2-d]pyrimidin-5-amine, which has a high inhibitory activity against CDK4 and CDK6, and weak inhibitory activity against CDK1 and CDK2, with obvious 4, 6 selectivity.

The disclosure also includes a pharmaceutically acceptable salt of the compound of formula X, a polymorph of the compound of formula X, or a polymorph of pharmaceutically acceptable salt of the compound of formula X.

In the present disclosure, the pharmaceutically acceptable salt is selected from the group consisting of hydrochloride, sulfate, hydrobromide, phosphate, methanesulfonate, maleate, L-tartrate, citrate, fumarate, and formate.

### Polymorphs

Solid exists in amorphous form or in crystalline form. In the case of crystal form, the molecules are localized in the three-dimensional lattice sites. When a compound is crystallized from a solution or slurry, it can be crystallized in different space lattice arrangement (this property is called "polymorphism") to form crystals with different crystalline forms, each of which is called as "polymorphs." Different polymorphs of a given substance may differ from each other in one or more physical properties (such as solubility and dissolution rate, true specific gravity, crystalline form, packing pattern, flowability and/or solid state stability).

### Crystallization

Crystallization on the production scale can be accomplished by manipulating a solution such that the solubility limit of the interested compound is exceeded. This can be done in a number of ways, for example, by dissolving the compound at a relatively high temperature and then cooling the solution below a saturation limit, or by boiling, evaporating at ordinary pressure, drying under vacuum or by some other means to reduce the liquid volume. The solubility of the interested compound may be reduced by adding an anti-solvent or a solvent in which the compound has a low solubility or a mixture of such solvents. An alternative method is to adjust the pH to reduce the solubility. See Crystallization, Third Edition, J W Mullens, Butterworth-Heineman Ltd., 1993, ISBN 0750611294 for a detailed description of crystallization.

The "suspension stirring" described in the present disclosure means a way to get crystals by mixing the compound of formula X with a corresponding acid or a solution of the corresponding acid to form a turbid solution, or by mixing the compound of formula X with a suitable solvent to form a turbid solution before stirring. Suitable solvents may be water or organic solvents.

The "slow volatilization" described in the present disclosure means a way to get crystals by placing a solution of the compound of formula X or a solution containing the compound of formula X and the corresponding acid at a certain temperature for slow volatilization of the solvent.

The "addition of anti-solvent" described in the present disclosure means a way to get crystals by adding a suitable solvent to a solution of the compound of formula X and precipitating the crystals.

If salt formation and crystallization are expected to occur at the same time, the addition of an appropriate acid or base can result in the direct crystallization of the desired salt if the salt has a lower solubility in the reaction medium than the raw material. Likewise, in a medium in which the solubility of the desired final form is lower than that of reactant, the final product can be directly crystallized when the synthetic reaction is completed.

Optimization of crystallization can include inoculation of the crystal of desired form as a seed into the crystallization medium. In addition, many crystallization methods include a combination of the above strategies. One example is to dissolve the interested compound in a solvent at a high temperature, followed by the addition of an anti-solvent with a suitable volume in a controlled manner so that the system is just below saturation level. At this moment, the seed of desired form (the integrity of the seed is kept) can be added and the system is cooled to complete the crystallization.

As used herein, the term "room temperature" generally means 4-30 °C, preferably 20 ± 5 °C.

### Polymorphs of the present disclosure

As used herein, the term "the polymorph of the present disclosure" includes the polymorph of the compound of formula X or its pharmaceutically acceptable salts (e.g. hydrochloride, maleate), or the polymorph of various solvates of the compound of formula X, and also includes different polymorphs of the same salt or solvate.
"the polymorph of the compound of formula X" and "the polymorph of the free base of the compound of formula X" can be used interchangeably.

Preferred polymorphs of the disclosure include (but are not limited to):
(i) crystal forms A, B (including B-1, B-2, B-3), C (including C-1, C-2), D, E, F, G, H (including H-1, H-2), J, K (including K-1, K-2, K-3, K-4) (crystal form of salt);
(ii) crystal forms I, II, III, IV (crystal form of the compound of formula X).

In the present disclosure, some crystal forms can be converted into each other, so the present disclosure also provides a method of converting some crystal forms into each other.

### Identification and properties of polymorphs

The properties of the polymorph of the compound of formula X were studied using the following various ways and instruments after its preparation in the present disclosure.

### X-ray powder diffraction

Methods of determining X-ray powder diffraction of the crystals are known in the art. For example, an X-ray powder diffractometer was used to obtain a pattern with a copper radiation target at a scanning speed of 2° per minute.

The polymorph of the compound of formula X of the present disclosure or the polymorph of the pharmaceutically acceptable salt of the compound of formula X of the present disclosure has a specific crystalline form and has specific characteristic peaks in an X-ray powder diffraction (XRPD) pattern.

### Differential scanning calorimetry

It is also called "differential scanning calorimetry analysis" (DSC) which is a technique that measures the relationship between energy difference between the measured substance and the reference substance and temperature during heating. The location, shape and number of peaks on the DSC pattern are related to the nature of the substance, and therefore can be used to qualitatively identify the substance. This method can be commonly used in the art to detect the phase transition temperature, glass transition temperature, reaction heat and other parameters of a substance.

### Pharmaceutical compositions of compound of formula X and their use

Generally, the compound of formula X of the present disclosure or a pharmaceutically acceptable salt thereof may form a suitable dosage form for administration with one or more pharmaceutically acceptable carriers. These dosage forms are suitable for oral, rectal, topical, intraoral administration, and other parenteral administration (e.g., subcutaneous, intramuscular, intravenous administration, etc.). For example, dosage forms suitable for oral administration include capsules, tablets, granules, syrups and the like. Compounds of the present disclosure contained in these formulations may be solid powders or granules; aqueous or non-aqueous liquid solutions or suspensions; water-in-oil or oil-in-water emulsions. Such dosage forms may be prepared with active compounds and one or more carriers or excipients through the conventional pharmacy methods. The above-mentioned carriers should be compatible with active compounds or other excipients. For solid formulations, conventional non-toxic carriers include, but not limited to mannitol, lactose, starch, magnesium stearate, cellulose, glucose, sucrose and the like. Carriers used for liquid preparations include water, saline, aqueous dextrose, ethylene glycol and polyethylene glycol. The active compounds may form a solution or suspension with the above-mentioned carriers.

The compositions of the present disclosure are formulated, quantified and administrated in a manner consistent with the practice of medicine. The "effective amount" of the administrated compound depends on the factors such as the specific disease to be treated, the individual being treated, the cause of diseases, the drug targets and the mode of administration, etc.

The present disclosure provides the uses of a pharmaceutically acceptable salt of the compound of formula X, or a polymorph of the compound of formula X or a polymorph of the pharmaceutically acceptable salt of the compound of formula X according to the first aspect of the present disclosure in the preparation of a CDK4/CDK6 inhibitor or a medicament for treating CDK4/CDK6 related diseases.

Preferably, the CDK4/CDK6 related diseases are cancer, abnormal cell proliferative diseases, infections, inflammatory disorders, autoimmune diseases, cardiovascular diseases, neurodegenerative diseases, radiation-induced hematopoietic toxic diseases, or a combination thereof.

Preferably, the cancer is breast cancer, ovarian cancer, prostate cancer, melanoma, brain tumor, esophageal cancer, stomach cancer, liver cancer, pancreatic cancer, colorectal cancer, lung cancer, kidney cancer, skin cancer, glioblastoma, neuroblastoma, sarcoma, liposarcoma, osteochondroma, osteoma, osteosarcoma, seminoma, testicular tumor, uterine cancer, head and neck tumor, multiple myeloma, malignant lymphoma, polycythemia vera, leukemia, thyroid tumors, ureteral tumor, bladder tumor, gallbladder cancer, cholangiocarcinoma, chorionic epithelial cancer or pediatric tumors, or any combination thereof.

Preferably, the breast cancer is HR-positive, HER2-negative advanced breast cancer.

As used herein, "therapeutically effective amount" refers to the amount that yields a function or activity to humans and/or animals and may be tolerated by humans and/or animals.

As used herein, "pharmaceutically acceptable carrier" refers to a non-toxic, inert, solid, semi-solid substance or liquid filler, diluent, encapsulating material or auxiliary formulation or any type of excipient that is compatible with the patient which is preferably a mammal and more preferably a human. It is suitable for delivering active agent to a target without stopping the activity of the agent.

As used herein, "patient" refers to an animal, preferably a mammal, and more preferably a human. The term "mammal" refers to a warm-blooded vertebrate mammal, including, for example, cat, dog, rabbit, bear, fox, wolf, monkey, deer, rat, pig and human.

As used herein, "treatment" refers to alleviating, delaying progression, attenuating, preventing, or maintaining an existing disease or disorder (eg, cancer). Treatment also includes curing one or more symptoms of the disease or disorder, preventing its development or alleviating to some extent.

The therapeutically effective amount of the pharmaceutical composition of the present disclosure or the pharmaceutically acceptable salt of the compound of formula X, or the polymorph of the compound of formula X or the polymorph of pharmaceutically acceptable salt of the compound of formula X contained in the pharmaceutical composition is preferably 0.1 mg- 5g/kg (body weight).

### The main advantages of the present disclosure are:

The inventor found that the polymorphs of the 2-cyclopropyl-N- (5-((4-ethylpiperazin-1-yl) methyl) pyridin-2-yl) -3-isopropyl-3,8-di hydroimidazo[4',5',4,5]cyclopenta[1,2-d] pyrimidin-5-amine free base and its salts also had good physical and chemical properties and outstanding related pharmacological activities, and was an ideal CDK4/CDK6 inhibitor.

### DETAILED DESCRIPTION OF THE INVENTION

The present disclosure will be further illustrated below with reference to the specific examples. It should be understood that these examples are only to illustrate the disclosure but not to limit the scope of the present disclosure. The experimental methods without specific conditions in the following embodiments are generally carried out according to conventional conditions, or in accordance with the conditions recommended by the manufacturer. Unless indicated otherwise, parts and percentage are calculated by weight.

### Reagents and Instruments

In the present disclosure, the structure and purity of the compounds are identified by nuclear magnetic resonance (¹HNMR) and, or LC-MS mass spectrometry (LC-MS). ¹HNMR BrukerAVANCE-400 NMR machine, with tetramethylsilane (TMS) as the internal standard. LC-MS: Agilent 1200 HPLC System, 6140 MS liquid-mass spectrometer (available from Agilent), column WatersX-Bridge, 150×4.6mm, 3.5µm. Preparative high performance liquid chromatography (pre-HPLC): Waters PHW007, column XBridge C18, 4.6*150mm, 3.5um.

ISCO Combiflash-Rf75 or Rf200 automatic eluting column instrument, and Agela 4 g, 12g, 20g, 40g, 80g, 120g of disposable silica gel column were used.

Yantai Huanghai HSGF254 or Qingdao GF254 silica gel plates was used as thin-layer silica gel plate, in thin layer chromatography (TLC), 0.15mm - 0.2mm silica gel plates were used for detection of reaction, 0.4mm - 0.5mm silica gel plates were used for separation and purification of products in TLC. Yantai Huanghai 200-300 mesh silica gel is generally used as a carrier. FCP200 - 300 mesh alkaline aluminum oxide is commonly used as a carrier in alkaline aluminum oxide column.

All examples were performed under nitrogen or argon atmosphere and the solution refers to the aqueous solution if without special explanation.

As used herein, DMF refers to dimethylformamide, DMSO refers to dimethylsulfoxide, THF refers to tetrahydrofuran, DIEA refers to N,N-diisopropylethylamine, EA refers to ethyl acetate, PE refers to petroleum ether, BINAP refers to (2R, 3S)-2,2'- bis diphenylphosphino-1,1'-binaphthyl, NBS refers to N-bromosuccinimide, NCS refers to N-chlorosuccinimide, Pd₂(dba)₃ refers to tris(dibenzylideneacetone)dipalladium, and Pd(dppf)Cl₂ refers to [1,1'-bis (diphenylphosphino) ferrocene] palladium dichloride.

Acetonitrile ACN, methanol MeOH, ethanol EtOH, isopropyl alcohol IPA, acetone ACE, ethyl acetate EA, methyl tert-butyl ether MTBE, tetrahydrofuran THF, water H₂O, 50% acetonitrile 50% ACN, dicyclohexyl [3,6 -dimethoxy-2 ', 4', 6'-triisopropyl [1,1'-biphenyl] -2-yl] phosphine Brettphos.

As used herein, room temperature refers to about 20 ± 5 °C.

### General method

X-ray powder diffraction: in the present disclosure, the powder X-ray diffraction patterns of the above crystal forms are obtained by a method known in the art, using a D8 ADVANCE X-ray powder diffraction analyzer. The instrument test conditions are shown in the following table:

| Parameter | XRPD(Reflection mode) |
|---|---|
| X-ray | Cu, kα, Kα1 (Å): 1.54056 |
| X-ray tube settings | 40 kV, 40 mA |
| divergence slit | automatic |
| monochromator | none |
| Scanning mode | continuous |
| Scanning range (°2Theta) | 4°-40° |
| Scanning step (°2Theta) | 0.013 |
| Scan time (minutes) | ∼7 |

In the powder X-ray diffraction pattern, the site of each peak was determined by 2θ(°). It should be understood that different instruments and/or conditions could result in slightly different data and changes in peak site and relative intensity. The division of the intensity of peaks only reflects the approximate size of peaks in each site. In the present disclosure, the highest diffraction peak of each crystalline form was taken as the base peak which was defined as I₀ with the relative intensity as 100%, (the peak of crystal form I with 2θ(°) value of 6.68 is the base peak, the peak of crystal form II with 2θ(°) value of 15.03 is the base peak, the peak of crystal form III with 2θ(°) value of 5.23 is the base peak, the peak of crystal form IV with 2θ(°) value of 5.25 is the base peak, the peak of crystal form A with 2θ(°) value of 18.06 is the base peak, the peak of crystal form B-1 with 2θ(°) value of 14.75 is the base peak, the peak of crystal form B-2 with 2θ(°) value of 4.56 is the base peak, the peak of crystal form B-3 with 2θ(°) value of 14.96 is the base peak, the peak of crystal form C-1 with 2θ(°) value of 13.72 is the base peak, the peak of crystal form C-2 with 2θ(°) value of 10.47 is the base peak, the peak of crystal form D with 2θ(°) value of 21.04 is the base peak, the peak of crystal form E with 2θ(°) value of 19.33 is the base peak, the peak of crystal form F with 2θ(°) value of 12.03 is the base peak, the peak of crystal form G with 2θ(°) value of 10.13 is the base peak, the peak of crystal form H-1 with 2θ(°) value of 22.84 is the base peak, the peak of crystal form H-2 with 2θ(°) value of 11.67 is the base peak, the peak of crystal form J with 2θ(°) value of 10.64 is the base peak, the peak of crystal form K-1 with 2θ(°) value of 12.08 is the base peak, the peak of crystal form K-2 with 2θ(°) value of 5.23 is the base peak, the peak of crystal form K-3 with 2θ(°) value of 5.27 is the base peak, the peak of crystal form K-4 with 2θ(°) value of 15.04 is the base peak), and other peaks had the ratio of their peak height to the peak height of base peak as the relative intensity I, I₀. The definition of the relative intensity of each peak was shown in the following table:

| Relative intensity I/I₀(%) | Definition |
|---|---|
| 50-100 | VS (very strong) |
| 25-50 | S (strong) |
| 10-25 | M (medium) |
| 1-10 | W (weak) |

The acid-base molar ratio of the salts of the present disclosure or their crystalline forms was determined by HPLC/ IC or ¹H NMR

High performance liquid chromatography: in the present disclosure, high performance liquid chromatography (HPLC) is collected on an Agilent 1260 HPLC.

TGA and DSC pattern: TGA and DSC pattern were collected on TA Q500/5000 thermogravimetric analyzer and TA Q200/2000 differential scanning calorimeter respectively. The instrument test conditions are shown in the following table:

| Parameter | TGA | DSC |
|---|---|---|
| Method | Linear warming | Linear warming |
| Sample tray | Platinum plate, open | Aluminum plate, gland |
| Temperature range | Room temperature - set temperature | 25 °C - set temperature |
| Scanning rate ( °C, min) | 10 | 10 |
| Protective gas | Nitrogen | Nitrogen |

The Dynamic Vapor Sorption (DVS) curve: acquired on the DVS Intrinsic of Surface Measurement Systems. The relative humidity at 25 ° C is corrected with the deliquescence points of LiCl, Mg (NO3)₂ and KCl. The instrument test conditions are shown in the following table:

| Parameters | Setting value |
|---|---|
| Temperature | 25 °C |
| Sample size | 10-20 mg |
| Protective gas and flow | N₂, 200 ml/min |
| Dm/dt | 0.002%/min |
| Minimum dm/dt balance time | 10 minutes |
| The maximum balance time | 180 minutes |
| RH range | 0%RH-90%RH-0%RH |
| RH gradient | 10%(0%RH-90%RH, 90%RH-0%RH) |
| | 5%(90%RH-95%RH, 95%RH-90%RH) |

It should be understood that other numerical values may be obtained when other types of instruments with the same function as the instruments described above or test conditions which are different from the conditions used in the present disclosure were used. Therefore, the recited value should not be considered as an absolute numerical value.

Due to the instrumental errors or different operators, one skilled in the art will understand that the above parameters used to characterize the physical properties of crystals may differ slightly, so the parameters described above are only used to assist in characterizing the polymorphs provided herein, and can not be regarded as a limitation on the polymorphs of the present disclosure.

### Intermediate preparation

### Preparation of compound 1a

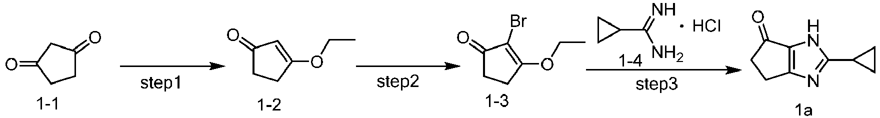

Step 1: compound 1-1 (50 g, 0.51 mol) was dissolved in 500ml of ethanol, compound p-toluenesulfonic acid was added, and refluxed for 48h. The reaction solution was cooled to room temperature, concentrated under reduced pressure, the solvent was removed, and then 500ml of ethanol was added, continued to reflux for 18h. After cooling to room temperature, the reaction solution was concentrated under reduced pressure. Most of the solvent was removed, filtered through celite and the filter cake was washed with ethanol. The filtrate was concentrated under reduced pressure to obtain the compound 1-2 (62 g), which was directly used in the next reaction. MS m/z(ESI): 127 [M+H]⁺
Step 2: compound 1-2 (62 g, 0.51 mol) was dissolved in 600 ml of dichloromethane, NBS (100 g, 0.56 mol) was added in portions at 5-10°C, and the reaction solution was stirred at room temperature for 16 h and concentrated under reduced pressure, After removing dichloromethane, 600 ml of methyl tert-butyl ether was added to the crude product, stirred, filtered, and the filtrate was concentrated to obtain the compound 1-3 (95 g), which was directly used in the next reaction. MS m/z(ESI): 206 [M+H]⁺.
Step 3: compound 1-3 (446 g, 2.175 mol) was dissolved in DMF (3L), and compound 1-4 (263.2 g, 2.175 mol), potassium carbonate (870 g, 6.30 mol) were added. The reaction solution was stirred at 100 °C for 2h, and quenched with saturated ammonium chloride solution. The solvent was removed under reduced pressure, and the crude product was purified by column chromatography and then slurried with ethyl acetate to obtain the compound 1a (195 g, yield 55%), MS m/z(ESI): 162 [M+H]⁺.

### Preparation of compound 2a

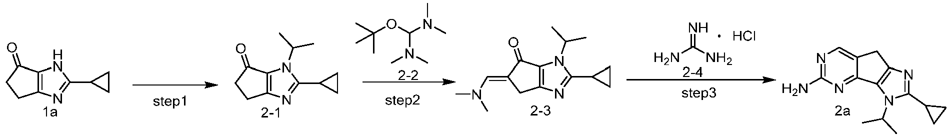

Step 1: compound 1a (10 g, 0.062 mol) was dissolved in DMF (100 ml), sodium hydrogen (3 g, 0.075 mol) was added in portions at 5-10 °C, the reaction solution was stirred at 5 °C for 1 h , iodoisopropane (7.5 ml, 0.75 mol) was slowly added, the reaction solution was stirred at 60 °C for 4h, and concentrated under reduced pressure to remove the solvent, and the crude product was purified by column chromatography to obtain compound 2-1 (2.5 g). MS m/z(ESI): 204 [M+H]⁺.
Step 2: compound 2-1 (30 g, 0.147 mol) and compound 2-2 (130 ml, 0.632 mol) were stirred at 110 °C for 4h. The reaction solution was concentrated under reduced pressure to obtain compound 2-3 (40 g), which was directly used in the next reaction. MS m/z(ESI): 260 [M+H]⁺.
Step 3: compound 2-3 (40 g, 0.147 mol), compound 2-4 (140.4 g, 1.47 mol), and sodium ethoxide (100 g, 1.47 mol) were dissolved in ethanol and stirred at 120 °C in a sealed tube for 36h. After the reaction solution was cooled to room temperature, the insoluble solid was removed by filtration, the filtrate was concentrated, and purified by column chromatography to obtain compound 2a (25g, yield 67%), MS m/z(ESI): 256 [M+H]⁺.

### Preparation of compound 3a

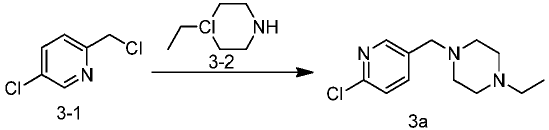

Compound 3-1 (142g, 0.877 mol), compound 3-2 (100g, 0.877 mol) and potassium carbonate (242 g, 1.754 mol) were dissolved in ethanol, the reaction solution was stirred at 60 °C for 24h. After the reaction solution was cooled to room temperature, the insoluble solid was removed by filtration, the filtrate was concentrated under reduced pressure, and purified by column chromatography to obtain compound 3a (59 g, yield 97%), MS m/z(ESI): 240 [M+H]⁺.

### Example 1 Preparation of compound of formula X

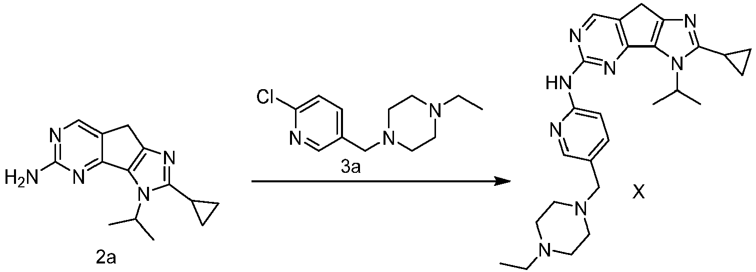

Compound 2a (1.5 g, 5.9 mmol), compound 3a (1.83g, 7.6 mmol), cesium carbonate (3.81g, 11.7 mmol), palladium acetate (66mg, 0.3 mmol) and Brettphos (0.16g, 0.3 mmol) were dissolved in diethylene glycol diethyl ether, and stirred overnight at 150 °C under nitrogen protection. After the reaction solution was cooled to room temperature, the reaction was quenched with methanol, insoluble solids were removed by filterring through celite, and the filtrate was concentrated. The crude product was purified by Pre-HPLC acid preparation column (the acid in the mobile phase was formic acid) to obtain the formate of compound of formula X (1.8g, yield 69%) as yellow powder, MS m/z(ESI): 459 [M+H]⁺, ¹H NMR (400 MHz, DMSO) δ 9.44 (s, 1H), 8.35-8.32 (m, 2H), 8.18-8.16 (m, 2H), 7.68 (dd, 1H), 4.95-4.92 (m, 1H), 3.44 (d, 4H), 2.49-2.39 (m, 8H), 2.35-2.34 (m, 2H), 2.32-2.30 (m, 1H), 1.68 (d, 6H), 1.04-0.94 (m, 7H). The obtained powder was sent for XRD detection, and its powder X-ray diffraction pattern showed no obvious characteristic peaks, so it was in amorphous form. The obtained formate of compound of formula X was converted into the free base form by Pre-HPLC (NH₃H₂O-NH₄HCO₃) to obtain 1.38 g of compound of formula X free base as a white solid.

### Example 2 Preparation of crystal form A of compound of formula X

800 mg of the free base of compound of formula X obtained according to the method of Example 1 was dissolved in 30 ml of acetone, and completely dispersed by ultrasound; 2100 µL of maleic acid (concentration 1M) was added, the acid-base molar ratio was 1.2: 1, stirred under magnetic force at 40 °C for 4 h. The reaction suspension was filtered by vacuum pump, rinsed, and dried in an oven at 40 °C to obtain an off-white to yellow powder, with a yield of 87.57%. The powder X-ray diffraction pattern of the obtained crystal is shown in Figure 1 (2θ angle has been marked), ¹HMR is as shown in Figure 28, the acid-base molar ratio is 1.2: 1, the melting point is 206 °C, and the crystal form is defined as crystal form A in this application.

### Example 3 Preparation of crystal form A of compound of formula X

15 mg of the free base of compound of formula X obtained according to the method of Example 1 was added into a sample bottle respectively, and 1-3 ml of ethanol, acetone, ethyl acetate or 50% acetonitrile was added to completely dissolve the compound, 0.039 ml of 1M maleic acid was added, a stirrer was added and the sample bottle was sealed, and the compound reacted with the acid under a water bath at 50 °C for 4 hours. The sample bottle was taken out, and placed in the refrigerator at 4 °C overnight after cooling to room temperature. The obtained turbid liquid of ethanol, acetone or ethyl acetate was centrifuged and the supernatant was poured, precipitated and dried by volatilization; the solution of the salt in 50% acetonitrile was placed in a fume hood for slow volatilization, thereby to obtain an off-white to yellow powder. The powder X-ray diffraction pattern of the obtained crystal is shown in Figure 1 (2θ angle has been marked), ¹HMR is as shown in Figure 28, the acid-base molar ratio is 1.2: 1, the melting point is 206 °C, and the crystal form is defined as crystal form A in this application.

### Example 4 Preparation of crystal form B-1 of compound of formula X

800 mg of the free base of compound of formula X obtained according to the method of Example 1 was dissolved in 30 ml of acetone or ethyl acetate, and completely dispersed by ultrasound; 2100 µL of hydrochloric acid (concentration 1M) was added, the acid-base molar ratio was 1.2: 1, the mixture was stirred under magnetic force at 40 °C for 4 h. The reaction suspension was filtered by vacuum pump, rinsed, and dried in an oven at 40 °C to obtain a light yellow powder, with a yield of 81.7%. The powder X-ray diffraction pattern of the obtained crystal is shown in Figure 4 (2θ angle has been marked), the acid-base molar ratio is 1.2: 1, and the crystal form is defined as crystal form B-1 in this application.

### Example 5 Preparation of crystal form B-2 of compound of formula X

20 mg of crystal form B-1 of compound of formula X was added into a 15ml centrifuge tube, 11ml of acetonitrile was added, the tube was sealed and placed under a water bath at 60 °C, heated to obtain a turbid liquid, the supernatant was added into another centrifuge tube, and stored in a refrigerator at 4 °C fot 2 -3 days, taken out for centrifugation and dried to obtain an off-white powder. The powder X-ray diffraction pattern of the obtained crystal is shown in Figure 7 (2θ angle has been marked), the acid-base molar ratio is 1.2: 1, and the crystal form is defined as crystal form B-2 in this application.

### Example 6 Preparation of crystal form B-3 of compound of formula X

20 mg of crystal form B-1 of compound of formula X was added into a 15ml centrifuge tube, 11ml of acetone was added, the tube was sealed and placed under a water bath at 60 °C, heated to dissolve, the dissolved sample was cooled to room temperature, and placed in a refrigerator at 4 °C fot 2 -3 days, taken out for centrifugation and dried to obtain an off-white powder. The powder X-ray diffraction pattern of the obtained crystal is shown in Figure 8 (2θ angle has been marked), the acid-base molar ratio is 1.2: 1, and the crystal form is defined as crystal form B-3 in this application.

### Example 7 Preparation of crystal form C-1 to crystal form J of compound of formula X

15 mg of the free base of compound of formula X obtained according to the method of Example 1 was added into a sample bottle respectively, and 3ml of the corresponding solvent was added to completely dissolve the compound, the acid at a certain volume and concentration was added, a stirrer was added and the sample bottle was sealed, and the solution was reacted under a water bath at 50 °C for 4 hours. The sample bottle was taken out, and placed in the refrigerator at 4 °C overnight after cooling to room temperature. The sample was centrifuged in a centrifuge (10000rmp, 10min), and the supernatant was poured, precipitated and placed in a fume hood for slow volatilization to obtain the corresponding crystals. The specific solvents and concentration and volume of the acids are shown in Table 1:

**Table 1**

| Acid | Concentration (mol/L) | Volume (ml) | ethanol | acetone | ethyl acetate | 50%acetonitrile |
|---|---|---|---|---|---|---|
| Sulfuric acid | 0.5 | 0.078 | Crystal form C-1 | Crystal form C-1 | Crystal form C-2 | |
| Hydrobromic acid | 1 | 0.039 | Amorphous | Amorphous | Crystal form D | |
| Phosphoric acid | 1 | 0.039 | Amorphous | Crystal form F | Amorphous | |
| Methanesulfonic acid | 1 | 0.039 | Crystal form J | Crystal form J | Crystal form J | Crystal form J |
| L-tartaric acid | 1 | 0.039 | Amorphous | Crystal form E | Amorphous | |
| Citric acid | 0.5 | 0.078 | Amorphous | Crystal form G | Amorphous | |
| Fumaric acid | 0.25 | 0.156 | Crystal form H-2 | Crystal form H-1 | Crystal form H-2 | |

### Example 9 Preparation of crystal form K-1, K-2 of the formate of compound of formula X

15 mg of the formate of compound of formula X obtained according to the method of Example 1 was weighed into centrifuge tubes respectively, 500 µL of solvent was added, if the formate was completely dissolved, the solution was placed in a fume hood and dried by volatilization at room temperature; and if the formate was not completely dissolved, the solution was heated in a water bath at 75 °C, and then cooled at room temperature if the formate was completely dissolved. If solid is precipitated, the precipitated solid was centrifuged and the supernatant and dried by volatilization, and if no solid is precipitated, the solution was placed in a fume hood and dried by volatilization at room temperature; if it was not dissolved when heated, the solution was shaken at 25 °C for 24h (at a speed of 170rpm) on a shaker, and then centrifuged to discard the supernatant, the remaining solid was dried by volatilization. The specific treatment and obtained crystal forms are shown in Table 2:

**Table 2**

| Solvent | Formate of compound of formula X | Crystal form | Solid form |
|---|---|---|---|
| Acetone | Heated to dissolve, no precipitation when cooling; slow volatilization | Crystal form K-2 | Off-white powder |
| Acetonitrile | Heated to dissolve, no precipitation when cooling; slow volatilization | Crystal form K-2 | Off-white powder |
| Ethyl acetate | Heated to dissolve, no precipitation when cooling; slow volatilization | Crystal form K-1 | Off-white to light yellow powder |
| Ethanol | Completely dissolved; slow volatilization | Crystal form K-1 | Off-white to light yellow powder |
| Isopropanol | Completely dissolved; slow volatilization | Crystal form K-1 | Off-white to light yellow powder |
| Methanol | Completely dissolved; slow volatilization | Crystal form K-1 | Off-white to light yellow powder |
| Tetrahydrofuran | Completely dissolved; slow volatilization | Crystal form K-1 | Off-white to light yellow powder |

### Example 10 Preparation of crystal form K-3 of the formate of compound of formula X

20 mg of the formate of compound of formula X obtained according to the method of Example 1 was added into a 15 ml centrifuge tube, and 6 ml of acetonitrile was added. The tube was sealed and placed under a water bath at 60 °C, heated to completely dissolve, the sample was cooled to room temperature, and stored in a refrigerator at 4 °C overnight, taken out for centrifugation and dried to obtain an off-white powder. The powder X-ray diffraction pattern of the obtained crystal is shown in Figure 20 (2θ angle has been marked), the acid-base molar ratio is 1.2: 1, and the crystal form is defined as crystal form K-3 in this application.

### Example 11 Preparation of crystal form K-4 of the formate of compound of formula X

20 mg of the formate of compound of formula X obtained according to the method of Example 1 was added into a 15 ml centrifuge tube, and 6.5 ml of acetone was added. The tube was sealed and placed under a water bath at 60 °C, heated to completely dissolve, the sample was cooled to room temperature, and stored in a refrigerator at 4 °C overnight, taken out for centrifugation and dried to obtain an off-white powder. The powder X-ray diffraction pattern of the obtained crystal is shown in Figure 21 (2θ angle has been marked), the acid-base molar ratio is 1.2: 1, and the crystal form is defined as crystal form K-4 in this application.

### Example 12 Preparation of crystal form I of free base of compound of formula X

The free base of the compound of formula X obtained according to the method of Example 1 was pulpified with diethylene glycol diethyl ether at room temperature for 4 hours, filtered, and the filter cake was washed with acetone, and then the filter cake was further pulpified with diethylene glycol diethyl ether at room temperature overnight, filtered, and the filter cake was washed with acetone and dried to obtain an off-white powder, the resulting powder X-ray diffraction pattern is shown in Figure 22 (2θ angle has been marked), the melting point is 199-120 °C, and the crystal form is defined as crystal form I of the free base of compound of formula X in this application.

### Example 13 Preparation of crystal form II-IV of free base of compound of formula X

10 mg of the free base of the compound of formula X obtained according to the method of Example 1 was weighed into a sample bottle, and 100 µL or 200 µL or 500 µL of solvent was gradually added to the bottle to completely dissolve or until 11 ml of solvent was added. The resulting solution or suspension was placed in a fume hood for slow volatilization until solids were precipitated. The experimental results are shown in Table 3:

**Table 3 crystal form of free base of compound of formula X**

| Solvent | The amount of solvent added in ml | Solution state | Crystallization | Crystal form |
|---|---|---|---|---|
| Acetonitrile | 11 | Heated to be clear | slow volatilization | Crystal form II |
| 50% Acetonitrile | 0.4 | Clear | slow volatilization | Crystal form II |
| Ethanol | 0.2 | Clear | slow volatilization | Crystal form II |
| Isopropanol | 0.5 | Clear | slow volatilization | Crystal form II |
| Acetone | 2 | Clear | slow volatilization | Crystal form II |
| Ethyl acetate | 1 | Clear | slow volatilization | Crystal form II |
| Methyl tert-butyl ether | 5 | Clear | slow volatilization | Crystal form III |
| Tetrahydrofuran | 0.1 | Clear | slow volatilization | Crystal form IV |
| N-heptane | 5 | Turbid | Suspension, Dried | Crystal form III |

### Example 14 Stability experiment

### 1. Stability experiment of crystal form A and crystal form B-1

(A) An appropriate amount of samples were weighed respectively and placed open at room temperature and under accelerated conditions (40 °C / 75% RH), on day 0, 7, 14, and 28
(B) Crystal form A was placed in an oven at 60 °C, on day 14, 32.

The content and related substance changes were detected by HPLC, and XRD was measured at the same time to investigate stability of the crystal forms.

**Table 4 results of content and related substance**

| Sample Name | Content(%) | Impurity peak |
|---|---|---|
| Crystal form B-1(on day 0) | 94.43 | none |
| Crystal form B-1 (room temperature, on day 7) | 96.50 | none |
| Crystal form B-1 (room temperature, on day 14) | 96.45 | none |
| Crystal form B-1 (room temperature, on day 28 *) | 107.12 | none |
| Crystal form B-1 (40°C, on day 7) | 94.36 | none |
| Crystal form B-1 (40°C, on day 14) | 95.19 | none |
| Crystal form B-1 (40°C, on day 28 *) | 110.05 | none |
| Crystal form A (on day 0) | 103.14 | none |
| Crystal form A (room temperature, on day 7) | 103.82 | none |
| Crystal form A (room temperature, on day 14) | 104.20 | none |
| Crystal form A (room temperature, on day 28*) | 116.27 | none |
| Crystal form A (40°C, on day 7) | 100.97 | none |
| Crystal form A (40°C, on day 14) | 104.46 | none |
| Crystal form A (40°C, on day 28*) | 113.75 | none |
| Crystal form A (60°C, on day 14) | 106.2 | none |
| Crystal form A (60°C, on day 32) | 104.5 | none |

| | | |
|---|---|---|
| * The content is obviously higher on day 28, and the measurement has errors | | |

Note: Crystal form B-1 will decompose and deacidify above 40 °C, and will not be included in the experiment at 60 °C.

The content of crystal form A and crystal form B-1 don't change significantly at room temperature and under accelerated conditions, no impurity peaks appear, and the chemical properties are stable.

The content of crystal form A and related substances have no obvious changes at 60 °C, and the chemical properties are stable.

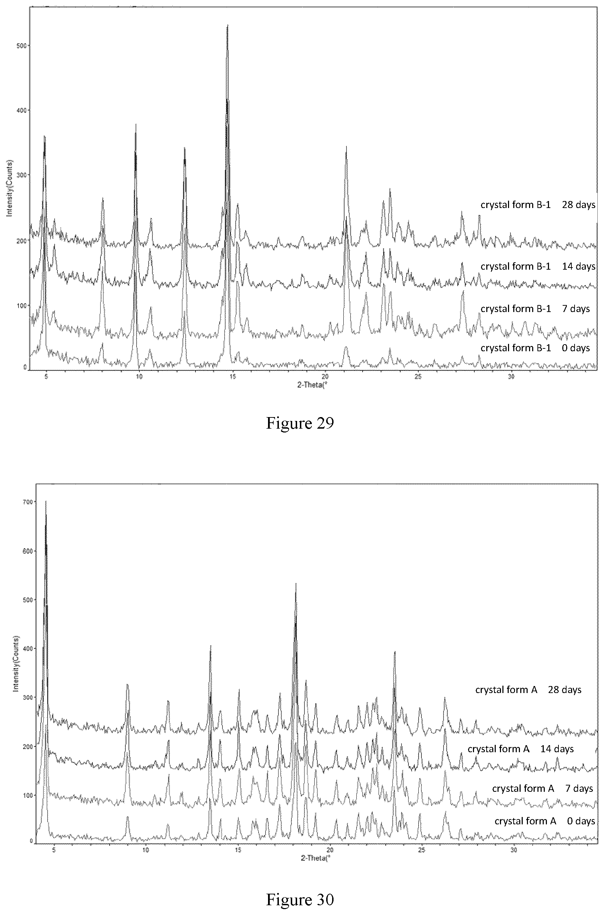

The characteristic peaks of the XRD pattern of crystal form A and crystal form B-1 have not changed under accelerated conditions for 28 days, and the crystal form is stable (as shown in Figure 29, 30).

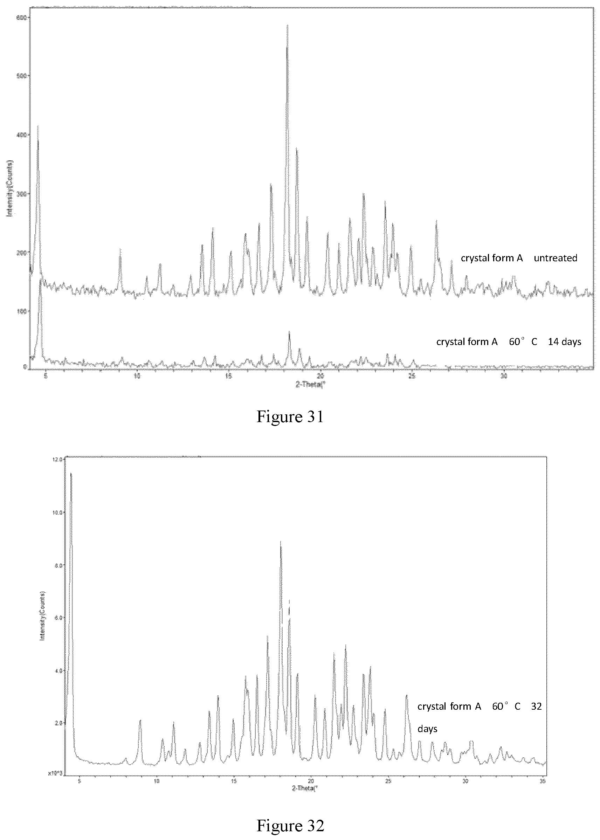

Crystal form A is not transformed at a high temperature of 60 °C for 14 days and 32 days, and the crystal form is stable (as shown in Figure 31, 32).

### Example 15 Solubility experiment

1) About 5-20 mg of crystal form I is weighed into centrifuge tube, and 200µL H₂O, 0.1M HCl, and medium (pH4.5, pH6.8 or pH7.4) were added respectively, the medium became turbid, and was centrifuged (1000Orpm, 10min). The supernatant was diluted 100 times into the liquid phase;
2) About 5 mg of crystal form A, crystal form B-1, crystal form K-1 were weighed into a centrifuge tube respectively. 200µL H₂O, 0.1M HCl, and aqueous mediums (pH4.5, pH6.8 or pH7.4) and seven organic solvents(i.e. acetone, acetonitrile, ethyl acetate, ethanol, methanol, isopropanol or tetrahydrofuran) were added respectively, and dissolved by ultrasound. After the compounds in some centrifuge tubes were completely dissolved, 5 mg-10 mg of crystal form A, crystal form B-1, and crystal form K-1 were continuously added again respectively to make the solution turbid. The solutions of crystal form K-1 in four organic solvents (i.e. acetonitrile, ethanol, isopropanol and methanol) and the above five aqueous mediums are clear; and the solutions of crystal form B-1 in the acetone and methanol are clear;
3) The clear solution was diluted 100 times and then entered into the liquid phase; the turbid liquid was centrifuged (10000rpm, 10min) and the supernatant was diluted 10-100 times into the liquid phase. The HPLC is shown in Table 5, and the experimental results are shown in Table 6:

**Table 5**

| | | | |
|---|---|---|---|
| HPLC | HY-ZL-HPLC006 | | |
| Mobile phase | A: 0.01% TFA in water | | |
| | B: 0.01% TFA in acetonitrile | | |
| Gradient | Time (min) | A | B |
| | 0 | 95 | 5 |
| | 9 | 5 | 95 |
| | 15 | 5 | 95 |
| | 15.1 | 95 | 5 |
| Column | XBridgeC18 5µm, 4.6*150mm | | |
| Flow rate (mL/min) | 1 | | |
| Wavelength (nm) | 254 | | |
| Injection volume (µL) | 5 | | |
| Column temperature (°C) | 30 | | |
| Retention time (min) | 15.1 | | |

**Table 6**

| Solubility in mg/ml(calculated for crvstal form I) | | | | |
|---|---|---|---|---|
| Solvent, medium | Crystal form I | Crystal form K-1 | Crystal form B-1 | Crystal form A |
| Acetonitrile | >1 | > 16.64 | 0.28 | 0.22 |
| 50% Acetonitrile | >25 | -- | -- | -- |
| Methanol | >100 | >30.41 | >45.19 | 41.04 |
| Ethanol | >50 | >41.86 | 19.59 | 6.97 |
| Isopropanol | >20 | >42.89 | 0.58 | 0.26 |
| Acetone | >5 | 11.47 | >41.11 | 0.23 |
| Ethyl acetate | >10 | 11.48 | 0.02 | 0.03 |
| Methyl tert-butyl ether | >2 | -- | -- | -- |
| Tetrahydrofuran | >100 | 39.09 | 0.39 | 0.15 |
| N-heptane | <2 | -- | -- | -- |
| Dimethyl sulfoxide | > 6.6 | -- | -- | -- |
| H₂O | 0.36 | >38.91 | >40.85 | 11.59 |
| 0.1 M HCl | 34.73 | >44.74 | >40.10 | >58.10 |
| pH4.5 | 9.66 | >35.09 | >29.40 | 14.30 |
| pH6.8 | 2.85 | >33.57 | >27.40 | 19.66 |
| pH7.4 | 1.08 | >38.78 | >25.65 | 17.86 |

As can be seen from the above table, (1) Crystal form I has relatively high solubility in 50% acetonitrile, methanol, ethanol, isopropanol, ethyl acetate and tetrahydrofuran, but lower solubility in acetonitrile, acetone, n-heptane and dimethyl sulfoxide, and is less soluble in aqueous medium (high solubility in 0.1M HCl); (2) crystal form K-1 has relatively high solubility in organic solvents and aqueous medium, which may be in a range of 11 to 45 mg/ml; (3) crystal form B-1 has is relatively high solubility in methanol, ethanol and acetone, but lower solubility in acetonitrile, isopropanol, tetrahydrofuran and ethyl acetate, and has relatively high solubility in aqueous medium, more than 25mg/ml; (3) crystal form A has relatively high solubility in methanol and ethanol; but lower solubility in other organic solvents and has relatively high solubility in aqueous medium, more than 11 mg/ml; in summary, crystal form K-1 has the best solubility, followed by crystal form B-2 and crystal form A, and crystal form I has the lowest solubility.

### Example 16 Hygroscopicity test

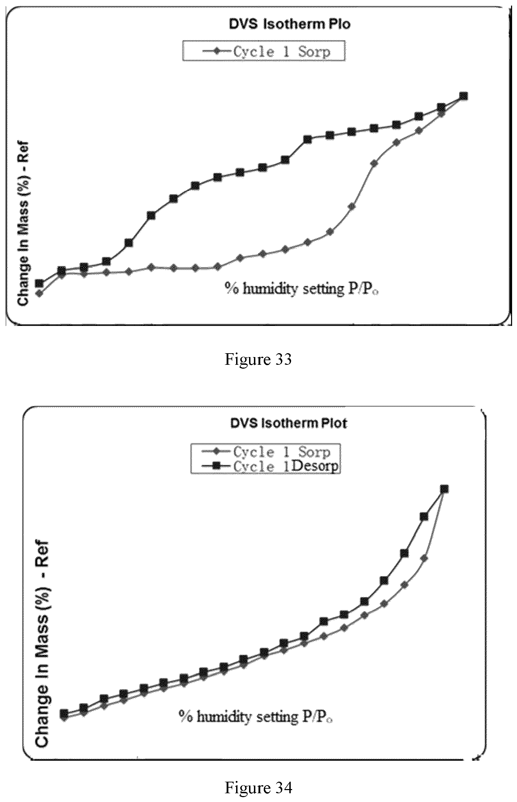

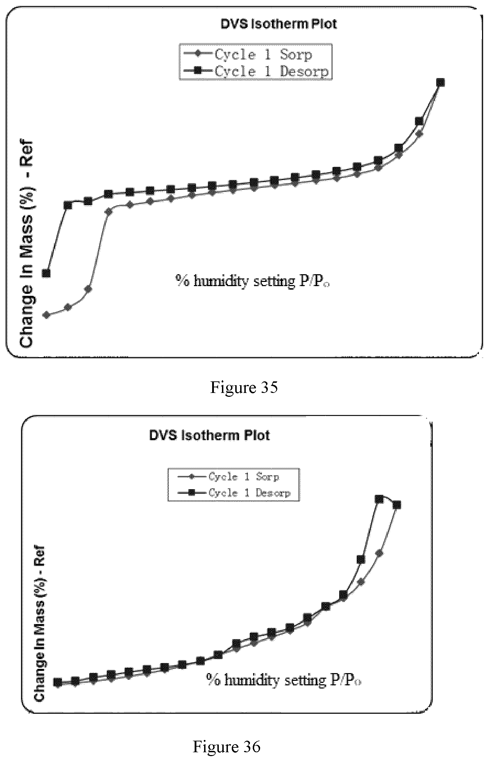

The DVS test results of crystal form I, crystal form A, crystal form B-1 and crystal form K-1 are shown in Figure 33-36, respectively, when the humidity increases to 80% RH at 25 °C, the hygroscopic weight gain of crystal form I is 8.84%, and has hygroscopicity; the hygroscopic weight gain of crystal form A is 0.613%, and is slightly hygroscopic; the hygroscopic weight gain of crystal form B-1 is 6.88, and has hygroscopicity. The hygroscopic weight gain of crystal form K-1 is 30.74% at 80% humidity, so this compound is a very hygroscopic drug.

### Example 17 Kinase test in vitro

Recombinant CDK1, CCNB1 and CDK9, and CCNT were purchased from BPS; CDK2, CCNA1, CDK4, CCND1 and CDK6, and CCND1 were purchased from Invitrogen; CDK4, CycD3 and CDK6, and CycD3 were purchased from Carna. Adenosine triphosphate (ATP) was purchased from Life tech. Substrate Ulight-4EBP1 and the corresponding detection antibody were purchased from Perkinelmer. LANCE Ultra system from Perkinelmer is used as the detection system.

In the kinase test, a compound to be tested was 1: 3 diluted for 8 gradient points and added into a reaction plate, and then an appropriate amount of recombinase was added. A buffer [50mM HEPES pH7.5, 10mM MgCl₂, 3mM MnCl₂, 1mM EGTA, 0.01% Tween-20, 1mM TCEP] containing ATP, Ulight-4EBP1 premix at a predetermined concentration was subsequently added, and the kinase reaction began at room temperature. A test solution pre-mixed with 10 mM EDTA and detection antibody was added after a suitable reaction time, and then the fluorescence value was read on Tecan infinite pro after reacting for 1 hour at room temperature. The IC₅₀ was calculated using a four-factor model fitting in a XLfit software. The results are shown in Table 7:

**Table 7 In vitro kinase activity of the compound of formula X**

| Compound | CDK4 IC₅₀(µM) | CDK6 IC₅₀(µM) | CDK1 IC₅₀(µM) | CDK2 IC₅₀(µM) |
|---|---|---|---|---|
| Free base of compound of formula X | 0.010 | 0.057 | 6.088 | 8.746 |
| Comparative compound 1 | 0.008 | 0.053 | 0.093 | 0.023 |

It can be seen from the above table that the compound of formula X has strong inhibitory activity against CDK4 and CDK6, but have weak inhibitory activity against CDK1 and CDK2, and therefore have a selective inhibitory activity against CDK4/CDK6. Although comparative compound 1 (the specific structure is shown below, and also can be seen in WO2012010704, Example 1-44) has a strong inhibitory activity against CDK4 and CDK6, it also has a strong inhibitory activity against CDK1 and CDK2, and therefore does not show selective inhibitory activity against CDK4/CDK6.

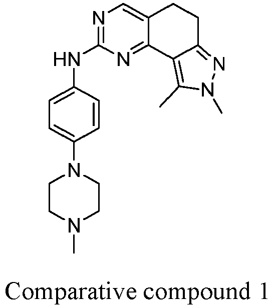

### Example 18 Pharmacokinetics and Brain Distribution Test

### Experimental Protocol:

Test Animals: healthy adult male SD rats (weight 210-230g per rat, 12 rats, fasted overnight, fed 4 hours after dosing), provided by SLAC company; preparation of oral solution: 40.14 mg of compound P-53 was weighed and added into a clean tube, and 36.479 mL of 0.5% HPC-H (TCI, E6ZQA) in acetate buffer (PH4.5) was added into the tube. The mixture was then vortexed for 1-2 min. Then the solution was sonicated for 20-25 min and stirred for 20-25 min.

SD rats were administered via intragastric administration (10 mg/kg (10 mL/kg)); samples were collected at 0.5, 1, 2 and 4 hr after dosing (total 4 timepoints), wherein only plasma samples were collected continuously, and brain tissue and cerebrospinal fluid were collected at each time point.

Blood collection: The animal was restrained manually and at each time point collected approximately 150 µL blood via tail vein into a K₂EDTA-containing tube. Within 15 minutes, the blood sample was put on wet ice and centrifuged to obtain plasma sample (2000g, 5 min under 4°C).

Brain tissue collection: an incision was made in the middle of the animal's scalp and then the skin was shrank. The skull was moved behind the brain by a small bone cutter and rongeurs. The brain was removed by a spatula, rinsed with cold saline, and placed in a screw-top tube, which was then stored under -70 °C until test.

Cerebrospinal fluid collection: The animal was euthanized under deep anesthesia with air bubble tail vein injection. The cerebrospinal fluid was collected by direct puncture of butterfly needle into the cisterna magna, using the occipital bone and the wings of the atlas as landmarks. A piece of white paper was used as a background just above the needle to monitor color change in the sampleduring collection. Upon observation of color change, the PE tube was quickly clamped off above the color change and cut just above the clamped site. And then the clear sample was drawn into the syringe.

Sample storage and possessing: Plasma, brain and cerebrospinal fluid samples were stored in dry ice temporarily and transferred into -80°C freezer for long term preservation.

Analytical method: the plasma and brain of SD rats were used as matrix, and Glipizide was used as an internal standard. LCMSMS-002 (API-4000, triple quadrupole) was used for testing and analysis. 30 µL of plasma samples and brain tissue samples were taken respectively and were added with 200 µL acetonitrile containing 100 ng/mL internal standard (Glipizide). The mixture was vortexed for 10 min and centrifuged at 5800 rpm for 10 min. 2 µL of centrifugated supernatant was taken for LC-MS/MS analysis. 10µL of MeOH/H₂O (1/1) and 60µL of acetonitrile containing 100 ng/mL internal standard (Glipizide) were added into 10 µL of cerebrospinal fluid sample. The mixture was vortexed for 5 min, and 2 µL supernatant was taken for LC-MS/MS analysis. The drug concentration was determined by LC-MS/MS method, the pharmacokinetic parameters in the plasma, brain tissue, and cerebrospinal fluid of rats are shown in Table 8:

**Table 8 Area under the curve after oral administration of 10 m/k of compound in rats**

| Compound | Free base of compound of formula X |
|---|---|
| AUC(hr*ng/mL) in plasma | 2620 |
| AUC(hr*ng/mL) in brain tissue | 4944 |
| AUC(hr*ng/mL) in cerebrospinal fluid | 204 |

As can be seen from Table 8, the compound of formula X can cross the blood-brain barrier and can be well distributed into the brain, which has better brain permeability.

### Example 19 Pharmaceutical composition

Tablet of crystal form A is prepared from the following components:

| | |
|---|---|
| Crystal form A | 15g |
| starch | 40g |
| lactose | 37g |
| PVP | 3g |
| Sodium hydroxymethyl starch | 3g |
| Sodium dodecyl sulfate | 1g |
| Magnesium stearate | 1g |

The crystal form A and starch are mixed and sieved, and then well mixed with the above other components, and tableted directly according to a conventional method.

### Example 20 Pharmaceutical composition

Capsule of crystal form I is prepared from the following components:

| | |
|---|---|
| Crystal form I | 20g |
| starch | 40g |
| lactose | 32g |
| PVP | 3g |
| Sodium hydroxymethyl starch | 3g |
| Sodium dodecyl sulfate | 1g |
| Magnesium stearate | 1g |

The crystal form I and starch are mixed and sieved, then well mixed with the above other components, and filled into ordinary transparent capsules according to a conventional method.

All publications mentioned herein are incorporated by reference as if each individual document was cited as a reference, as in the present application. It should also be understood that, after reading the above teachings of the present disclosure, those skilled in the art can make various changes or modifications, equivalents of which falls in the scope of claims as defined in the appended claims.

## Claims

1. A pharmaceutically acceptable salt of the compound of formula X and a polymorph thereof, 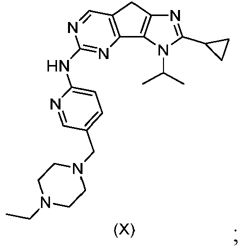 wherein the pharmaceutically acceptable salt is selected from the group consisting of hydrochloride, sulfate, hydrobromide, phosphate, methanesulfonate, maleate, L-tartrate, citrate, fumarate and formate.

2. The pharmaceutically acceptable salt of the compound of formula X and the polymorph thereof according to claim 1, wherein the pharmaceutically acceptable salt is selected from the group consisting of hydrochloride, maleate and formate.

3. The pharmaceutically acceptable salt of the compound of formula X and the polymorph thereof according to claim 1, wherein the polymorph is selected from the group consisting of
A crystalline form of the maleate of compound of formula X, i.e. crystal form A, the X-ray powder diffraction pattern of which has peaks at diffraction angles 2θ(°) values of the following group A1: 4.47±0.2, 8.93±0.2, 13.41±0.2, 13.98±0.2, 15.77±0.2, 16.52±0.2, 17.18±0.2, 18.06±0.2, 18.61±0.2, 19.16±0.2, 21.50±0.2, 22.26±0.2, 23.43±0.2, and 23.84±0.2;
B-1 crystalline form of the hydrochloride of compound of formula X, i.e. crystal form B-1, the X-ray powder diffraction pattern of which has peaks at diffraction angles 2θ(°) values of the following group B-1-1: 4.93±0.2, 6.78±0.2, 8.04±0.2, 9.82±0.2, 12.46±0.2, 14.75±0.2, 15.32±0.2, and 21.17±0.2;
B-2 crystalline form of the hydrochloride of compound of formula X, i.e. crystal form B-2, the X-ray powder diffraction pattern of which has peaks at diffraction angles 2θ(°) values of the following group B-2-1: 4.56±0.2, 11.41±0.2, and 13.60±0.2;
B-3 crystalline form of the hydrochloride of compound of formula X, i.e. crystal form B-3, the X-ray powder diffraction pattern of which has peaks at diffraction angles 2θ(°) values of the following group B-3-1: 5.03±0.2, 9.97±0.2, and 14.96±0.2;
C-1 crystalline form of the sulfate of compound of formula X, i.e. crystal form C-1, the X-ray powder diffraction pattern of which has peaks at diffraction angles 2θ(°) values of the following group C-1-1: 9.13±0.2, 9.71±0.2, 10.50±0.2, 11.19±0.2, 13.72±0.2, 13.94±0.2, 15.70±0.2, 16.79±0.2, 22.46±0.2, and 23.87±0.2;
C-2 crystalline form of the sulfate of compound of formula X, i.e. crystal form C-2, the X-ray powder diffraction pattern of which has peaks at diffraction angles 2θ(°) values of the following group C-2-1: 10.47±0.2, 14.78±0.2, and 15.72±0.2;
D crystalline form of the hydrobromide of compound of formula X, i.e. crystal form D, the X-ray powder diffraction pattern of which has peaks at diffraction angles 2θ(°) values of the following group D1: 7.99±0.2, 9.74±0.2, 10.53±0.2, 12.37±0.2, 14.64±0.2, 15.21±0.2, 21.04±0.2, 22.11±0.2, 23.03±0.2, 23.38±0.2, 24.45±0.2, and 27.22±0.2;
E crystalline form of the L-tartrate of compound of formula X, i.e. crystal form E, the X-ray powder diffraction pattern of which has peaks at diffraction angles 2θ(°) values of the following group E1: 6.43±0.2, 10.02±0.2, 11.63±0.2, 16.07±0.2, 19.33±0.2, 22.59±0.2, and 25.88±0.2;
F crystalline form of the phosphate of compound of formula X, i.e. crystal form F, the X-ray powder diffraction pattern of which has peaks at diffraction angles 2θ(°) values of the following group F1: 12.03±0.2, 17.26±0.2, and 19.65±0.2;
G crystalline form of the citrate of compound of formula X, i.e. crystal form G, the X-ray powder diffraction pattern of which has peaks at diffraction angles 2θ(°) values of the following group G1: 9.13±0.2, 10.13±0.2, 11.06±0.2, 12.38±0.2, 13.04±0.2, 14.07±0.2, 14.72±0.2, 15.33±0.2, 19.16±0.2, 20.31±0.2, 24.83±0.2, and 28.04±0.2;
H-1 crystalline form of the fumarate of compound of formula X, i.e. crystal form H-1, the X-ray powder diffraction pattern of which has peaks at diffraction angles 2θ(°) values of the following group H-1-1: 5.37±0.2, 10.74±0.2, 17.67±0.2, 19.08±0.2, 19.35±0.2, 20.11±0.2, 21.25±0.2, and 22.84±0.2;
H-2 crystalline form of the fumarate of compound of formula X, i.e. crystal form H-2, the X-ray powder diffraction pattern of which has peaks at diffraction angles 2θ(°) values of the following group H-2-1: 5.69±0.2, 11.67±0.2, 14.39±0.2, 21.15±0.2, and 23.49±0.2;
J crystalline form of the methanesulfonate of compound of formula X, i.e. crystal form J, the X-ray powder diffraction pattern of which has peaks at diffraction angles 2θ(°) values of the following group J1: 10.64±0.2, 18.70±0.2, 20.55±0.2, 20.86±0.2, 21.58±0.2, 22.16±0.2, 23.05±0.2, 24.39±0.2, 24.75±0.2, and 27.18±0.2;
K-1 crystalline form of the formate of compound of formula X, i.e. crystal form K-1, the X-ray powder diffraction pattern of which has peaks at diffraction angles 2θ(°) values of the following group K-1-1: 4.80±0.2, 8.43±0.2, 9.63±0.2, 9.88±0.2, 12.08±0.2, 13.87±0.2, 14.63±0.2, 18.02±0.2, 19.44±0.2, 20.05±0.2, 20.64±0.2, 22.47±0.2, and 23.68±0.2;
K-2 crystalline form of the formate of compound of formula X, i.e. crystal form K-2, the X-ray powder diffraction pattern of which has peaks at diffraction angles 2θ(°) values of the following group K-2-1: 5.23±0.2, 17.05±0.2, 17.31±0.2, 21.28±0.2, and 22.50±0.2;
K-3 crystalline form of the formate of compound of formula X, i.e. crystal form K-3, the X-ray powder diffraction pattern of which has peaks at diffraction angles 2θ(°) values of the following group K-3-1: 4.51±0.2, 5.27±0.2, and 13.76±0.2;
K-4 crystalline form of the formate of compound of formula X, i.e. crystal form K-4, the X-ray powder diffraction pattern of which has peaks at diffraction angles 2θ(°) values of the following group K-4-1: 13.26±0.2, 15.04±0.2, 16.18±0.2, 19.09±0.2, and 21.54±0.2;
crystal form I of the compound of formula X, the X-ray powder diffraction pattern of which has peaks at diffraction angles 2θ(°) values of the group 1-1: 6.20±0.2, 6.68±0.2, 13.42±0.2, 21.31±0.2, and 22.56±0.2;
crystal form II of the compound of formula X, the X-ray powder diffraction pattern of which has peaks at diffraction angles 2θ(°) values of the group II-1: 13.27±0.2, 15.03±0.2, 16.20±0.2, 19.09±0.2, and 21.56±0.2;
crystal form III of the compound of formula X, the X-ray powder diffraction pattern of which has peaks at diffraction angles 2Θ(°) values of the group III-1: 5.23±0.2 and 17.02±0.2; and
crystal form IV of the compound of formula X, the X-ray powder diffraction pattern of which has peaks at diffraction angles 2θ(°) values of the group IV-1: 5.25±0.2, 11.94±0.2, 12.23±0.2, 14.42±0.2, and 16.65±0.2.

4. The pharmaceutically acceptable salt of the compound of formula X and the polymorph thereof according to claim 3, wherein
the X-ray powder diffraction pattern of the crystal form A is substantially characterized as in Figure 1;
the X-ray powder diffraction pattern of the crystal form B-1 is substantially characterized as in Figure 4;
the X-ray powder diffraction pattern of the crystal form B-2 is substantially characterized as in Figure 7;
the X-ray powder diffraction pattern of the crystal form B-3 is substantially characterized as in Figure 8;
the X-ray powder diffraction pattern of the crystal form C-1 is substantially characterized as in Figure 9;
the X-ray powder diffraction pattern of the crystal form C-2 is substantially characterized as in Figure 10;
the X-ray powder diffraction pattern of the crystal form D is substantially characterized as in Figure 11;
the X-ray powder diffraction pattern of the crystal form E is substantially characterized as in Figure 12;
the X-ray powder diffraction pattern of the crystal form F is substantially characterized as in Figure 13;
the X-ray powder diffraction pattern of the crystal form G is substantially characterized as in Figure 14;
the X-ray powder diffraction pattern of the crystal form H-1 is substantially characterized as in Figure 15;
the X-ray powder diffraction pattern of the crystal form H-2 is substantially characterized as in Figure 16;
the X-ray powder diffraction pattern of the crystal form J is substantially characterized as in Figure 17;
the X-ray powder diffraction pattern of the crystal form K-1 is substantially characterized as in Figure 18;
the X-ray powder diffraction pattern of the crystal form K-2 is substantially characterized as in Figure 19;
the X-ray powder diffraction pattern of the crystal form K-3 is substantially characterized as in Figure 20; and
the X-ray powder diffraction pattern of the crystal form K-4 is substantially characterized as in Figure 21.

5. The pharmaceutically acceptable salt of the compound of formula X and the polymorph thereof according to claim 3, wherein
the X-ray powder diffraction pattern of the crystal form I is substantially characterized as in Figure 22;
the X-ray powder diffraction pattern of the crystal form II is substantially characterized as in Figure 25;
the X-ray powder diffraction pattern of the crystal form III is substantially characterized as in Figure 26; and
the X-ray powder diffraction pattern of the crystal form IV is substantially characterized as in Figure 27.

6. A process for preparing the pharmaceutically acceptable salt of the compound of formula X and the polymorph thereof, wherein the process comprises:
(1) reacting compound 2a with compound 3a in a solvent thereby to form the compound of formula X; 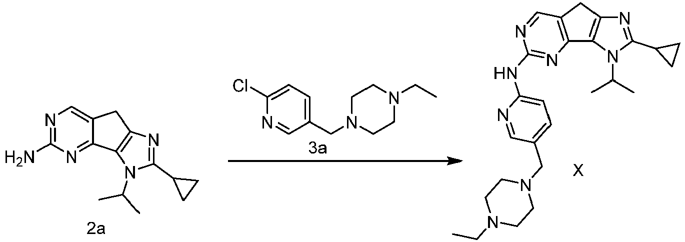
(2) optionally, performing a salt-forming reaction using the compound of formula X and an acid thereby to form a pharmaceutically acceptable salt; amd
(3) optionally, crystallizing the compound of formula X formed in step (1), or a pharmaceutically acceptable salt thereof formed in step (2) thereby to obtain a polymorph.

7. A pharmaceutical composition, wherein the pharmaceutical composition includes:
(a) the pharmaceutically acceptable salt of the compound of formula X and the polymorph thereof according to claim 1; and
(b) a pharmaceutically acceptable carrier.

8. Use of the pharmaceutically acceptable salt of the compound of formula X and the polymorph thereof according to claim 1, or the pharmaceutical composition according to claim 7 in the preparation of a drug for the treatment of a disease or disorder, the disease or disorder is selected from the group consisting of cancer, abnormal cell proliferative diseases, infections, inflammatory disorders, autoimmune diseases, cardiovascular diseases, neurodegenerative diseases, radiation-induced hematopoietic toxic diseases, or a combination thereof.

9. The use according to claim 8, wherein the cancer is selected from the group consisting of breast cancer, ovarian cancer, prostate cancer, melanoma, brain tumor, esophageal cancer, stomach cancer, liver cancer, pancreatic cancer, colorectal cancer, lung cancer, kidney cancer, skin cancer, glioblastoma, neuroblastoma, sarcoma, liposarcoma, osteochondroma, osteoma, osteosarcoma, seminoma, testicular tumor, uterine cancer, head and neck tumor, multiple myeloma, malignant lymphoma, polycythemia vera, leukemia, thyroid tumor, ureteral tumor, bladder tumor, gallbladder cancer, cholangiocarcinoma, chorionic epithelioma and pediatric tumor.

10. A method of inhibiting CDK4 and/or CDK6 activity, comprising administering to a patient in need thereof a therapeutically effective amount of the pharmaceutically acceptable salt of the compound of formula X and the polymorph thereof according to claim 1, or the pharmaceutical composition according to claim 7.
